# EUROPEAN PATENT APPLICATION

(11) **EP 4 775 583 A1**
(43) Date of publication of application: **15.07.2026**
(21) Application number: 24862815.8
(22) Date of filing: 04.09.2024
(51) Int. Cl.: C07D 487/04, A61K 31/519, A61P 43/00

(54) **COMPOUND OR SALT THEREOF, METHOD FOR PRODUCING SAME, PHARMACEUTICAL COMPOSITION CONTAINING COMPOUND OR SALT THEREOF, AND METHOD FOR PRODUCING SAME**

(30) Priority: 05.09.2023 JP 2023143583
(71) Applicant: Celaid Therapeutics Inc., Tokyo 113-8485 (JP); The University of Osaka, Osaka 565-0871 (JP)
(72) Inventor: SUZUKI, Takayoshi, Suita-shi, Osaka 565-0871 (JP); ITOH, Yukihiro, Suita-shi, Osaka 565-0871 (JP); WATANABE, Motoo, Tokyo 113-8485 (JP)
(74) Representative: Germain Maureau
(86) International application number: PCT/JP2024/031665
(87) International publication number: WO 2025/053163

(57) **Abstract**

A compound represented by the following formula (I) or a salt thereof. 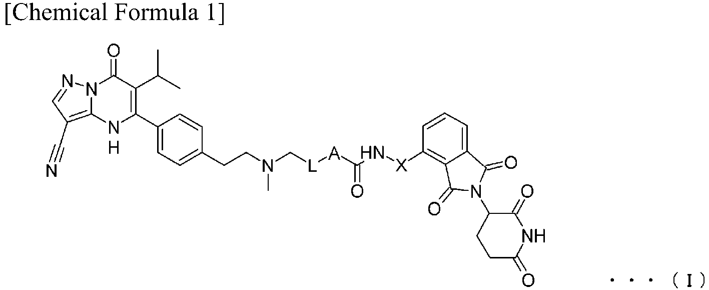 In Formula (I), A is a single bond or a substituted or unsubstituted divalent aromatic hydrocarbon group; L is a substituted or unsubstituted linear or branched alkylene group having 1 to 13 carbon atoms; and X is -(CH₂CH₂O)ₛ-, -(CH₂CH₂CH₂O)ₛ-, -(CH(CH₃)CH₂O)ₛ-, or -(CH₂)ₜO-, s is a number from 1 to 8 and t is a number from 2 to 10.

## Description

### TECHNICAL FIELD

The present disclosure relates to a compound or salt thereof and a method for producing the same, a pharmaceutical composition comprising the compound or salt thereof and a method for producing the same.

Priority is claimed on Japanese Patent Application No. 2023-143583, filed September 5, 2023, the content of which is incorporated herein by reference.

### BACKGROUND ART

Epigenetic modifications such as methylation and acetylation of histone proteins, control gene expression to regulate various life processes. Abnormalities in these modifications can lead to cancer, neurological disorders, and other diseases. To counter these abnormalities and the diseases they cause, epigenetic modification by small molecules is emerging as a promising therapeutic strategy.

Lysine demethylase 5 (KDM5) family proteins (KDM5A-D) which are Fe(II)/α-ketoglutarate-dependent oxidases, oxidatively remove methyl groups from tri- or dimethyl lysine 4 of histone H3 (H3K4me3/2). In addition to their enzymatic activities, KDM5s work as scaffold proteins, recruiting several repressive transcription factors including histone deacetylase 1 (HDAC1) and HDAC2. In other words, KDM5s epigenetically regulate gene expression in cooperation with other proteins rather than independently. Furthermore, KDM5s have been implicated in the development of various forms of cancer, drug resistance, and neurodegenerative disorders such as Alzheimer's disease.

Several KDM5 inhibitors have been identified to date.

Non-patent Document 1 describes a compound (KDM5- C49) represented by the following formula as a KDM5 inhibitor.

Non-Patent Document 2 describes a compound (CPI-455) represented by the following formula as a KDM5 inhibitor.

Non-Patent Document 3 describes a compound (S1) represented by the following formula as a KDM5 inhibitor.

Non-Patent Document 4 describes a compound (S2) represented by the following formula as a KDM5 inhibitor.

Non-Patent Document 5 describes a compound (S3) represented by the following formula as a KDM5 inhibitor.

Non-Patent Document 6 describes a compound (TK-129) represented by the following formula as a KDM5 inhibitor.

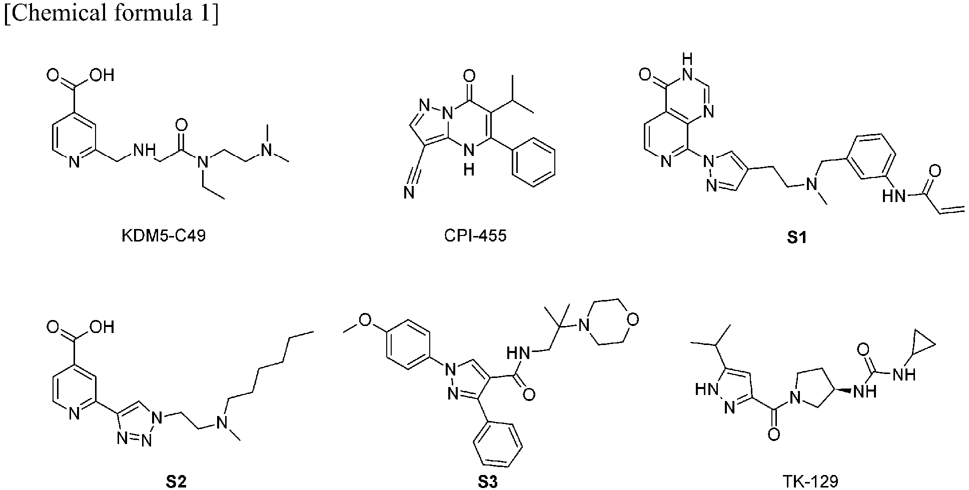

However, these KDM5 inhibitors have failed to produce any significant pharmacological effects, such as anti-cancer effects, in cells and animal studies.

### Citation List

### Non Patent Document

Non Patent Document 1: Johansson C., Velupillai S., Tumber A., Szykowska A., Hookway E.S., Nowak R.P., Strain-Damerell C., Gileadi C., Philpott M., Burgess-Brown N., Wu N., Kopec J., Nuzzi A., Steuber H., Egner U., Badock V., Munro S., LaThangue N.B., Westaway S., Brown J., Athanasou N., Prinjha R., Brennan P.E., Oppermann U., Nat. Chem. Biol., 12, 539-545 (2016).
Non-Patent Document 2: Vinogradova M., Gehling V.S., Gustafson A., Arora S., Tindell C.A., Wilson C., Williamson K.E., Guler G.D., Gangurde P., Manieri W., Busby J., Flynn E.M., Lan F., Kim H.J., Odate S., Cochran A.G., Liu Y., Wongchenko M., Yang Y., Cheung T.K., Maile T.M., Lau T., Costa M., Hegde G.V., Jackson E., Pitti R., Arnott D., Bailey C., Bellon S., Cummings R.T., Albrecht B.K., Harmange J.C., Kiefer J.R., Trojer P., Classon M., Nat. Chem. Biol. 12, 531-538 (2016).
Non-Patent Document 3: Vazquez-Rodriguez S., Wright M., Rogers C.M., Cribbs A.P., Velupillai S., Philpott M., Lee H., Dunford J.E., Huber K.V.M., Robers M.B., Vasta J.D., Thezenas M.L., Bonham S., Kessler B., Bennett J., Fedorov O., Raynaud F., Donovan A., Blagg J., Bavetsias V., Oppermann U., Bountra C., Kawamura A., Brennan P.E., Angew. Chem. Int. Ed. Engl., 58, 515-519 (2019).
Non-Patent Document 4: Miyake Y., Itoh Y., Suzuma Y., Kodama H., Uchida S., Suzuki T., ACS Catal., 10, 5383-5392 (2020).
Non-Patent Document 5: Zhao B., Liang Q., Ren H., Zhang X., Wu Y., Zhang K., Ma L.Y., Zheng Y.C., Liu H.M., Eur. J Med. Chem., 192, 112161 (2020).
Non-Patent Document 6: Tang K., Jiao L.M., Qi Y.R., Wang T.C., Li Y.L., Xu J.L., Wang Z.W., Yu B., Liu H.M., Zhao W., J. Med. Chem., 65, 12979-13000 (2022).

### SUMMARY OF INVENTION

### Technical Problem

The present disclosure was made to solve the above problems, and to provide compounds or salts thereof that produce remarkable pharmacological effects, such as anti-cancer effects, in cells or animal studies.

### Solution to Problem

The present inventors have conducted intensive studies to solve the above problems and, in order to identify a compound or a salt thereof that exhibits more potent pharmacological activity, focused on "proteolysis targeting chimeras (hereinafter, also referred to as PROTACs)," which degrade the target protein and inhibit its entire function. PROTACs are small molecules composed of two linked ligands. Based on the KDM5 inhibitors identified to date, novel candidate KDM5 PROTAC compounds were designed and synthesized. As a result of cell-based assays, it was revealed that the compounds or salts thereof of the present disclosure exhibit significant neurite outgrowth-promoting activity in neuroblastoma neuro-2a cells via the degradation of KDM5A. These results suggest that KDM5 PROTACs are promising drug candidates for the treatment of neurological diseases. The present inventors have found that the above problems can be solved by using the compounds or salts thereof of the present disclosure, and have thus completed the present disclosure.

That is, the gist of the present disclosure resides in the following [1] to [10].
[1] A compound represented by the following formula (I) 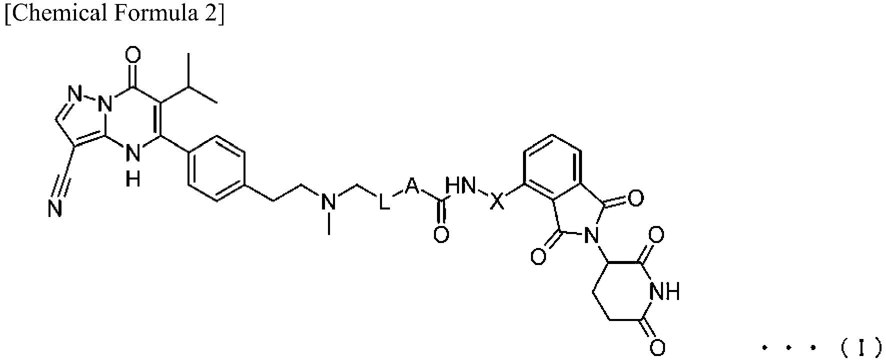 or a salt thereof, wherein, A is a single bond or a substituted or unsubstituted divalent aromatic hydrocarbon group; L is a substituted or unsubstituted linear or branched alkylene group having 1 to 13 carbon atoms; and X is -(CH₂CH₂O)ₛ-, -(CH₂CH₂CH₂O)ₛ-, -(CH(CH₃)CH₂O)ₛ-, or -(CH₂)ₜO-, wherein s is a number from 1 to 8 and t is a number from 2 to 10.
[2] The compound or a salt thereof according to [1], wherein, in said formula (I): A is a single bond or a phenylene group; L is a linear alkylene group having 3 to 10 carbon atoms; and X is - (CH₂CH₂O)ₛ-, -(CH₂CH₂CH₂O)ₛ-, -(CH(CH₃)CH₂O)ₛ-, or -(CH₂)ₜO-, wherein s is a number from 1 to 5 and t is a number from 2 to 10.
[3] The compound or salt thereof according to [1] or [2], wherein, in said formula (I): A is a single bond, p-phenylene group, or m-phenylene group; L is a linear alkylene group having 3 to 10 carbon atoms; and X is -(CH₂CH₂O)ₛ-, -(CH₂CH₂CH₂O)ₛ-, or -(CH₂)ₜO-, wherein s is a number from 1 to 5 and t is a number from 2 to 10.
[4] The compound or salt thereof according to any one of [1] to [3], wherein said compound is a compound represented by the following formula (I-1), a compound represented by the following formula (I-2), or a compound represented by the following formula (I-3): 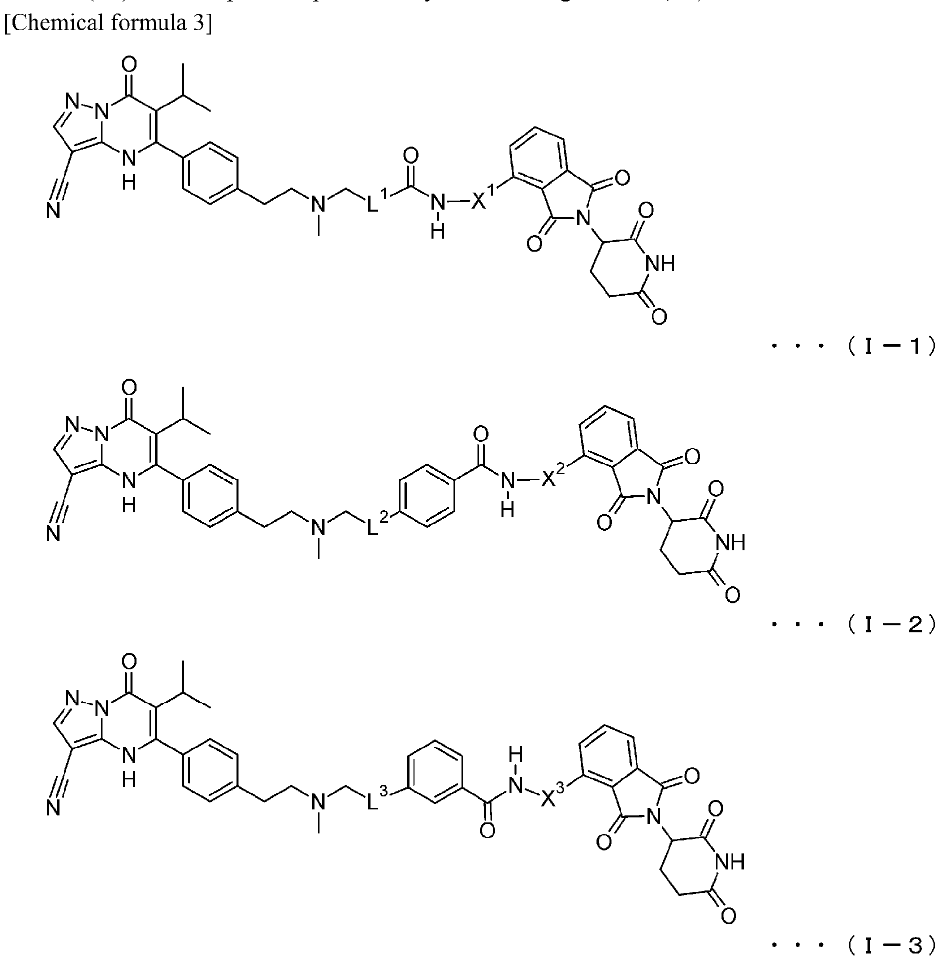 wherein: in formula (I-1), L¹is a linear alkylene group having 3 to 10 carbon atoms, and X¹ is a group represented by -(CH₂CH₂O)ₛ-, wherein s is a number from 1 to 5; in formula (I-2), L² is a linear alkylene group having 3 to 10 carbon atoms, and X² is a group represented by -(CH₂CH₂O)ₛ- or - (CH₂)ₜO-, wherein s is a number from 1 to 5 and t is a number from 2 to 10; and in formula (I-3), L³ is a linear alkylene group having 3 to 10 carbon atoms, and X³ is a group represented by -(CH₂CH₂O)ₛ- or -(CH₂)ₜO-, wherein s is a number from 1 to 5 and t is a number from 2 to 10.
[5] The compound or salt thereof according to any one of [1] to [4], wherein said compound is:
   a compound of said formula (I-1), wherein L¹ is a linear alkylene group having 5 carbon atoms, and X¹ is a group represented by -(CH₂CH₂O)ₛ-, and s is 3;
   a compound of said formula (I-1), wherein L¹ is a linear alkylene group having 8 carbon atoms, and X¹ is a group represented by -(CH₂CH₂O)ₛ-, and s is 3;
   a compound of said formula (I-2), wherein L² is a linear alkylene group having 5 carbon atoms, and X² is a group represented by -(CH₂CH₂O)ₛ-, and s is 3;
   a compound of said formula (I-2), wherein L² is a linear alkylene group having 5 carbon atoms, and X² is a group represented by -(CH₂)ₜO-, and t is 6;
   a compound of said formula (I-3), wherein L³ is a linear alkylene group having 5 carbon atoms, and X³ is a group represented by -(CH₂CH₂O)ₛ-, and s is 3; or
   a compound of said formula (I-3), wherein L³ is a linear alkylene group having 5 carbon atoms, and X³ is a group represented by -(CH₂)ₜO-, and t is 6.
[6] A pharmaceutical composition comprising the compound or salt thereof according to any one of [1] to [5].
[7] The pharmaceutical composition according to [6], which is a KDM5 inhibitor and a KDM5 degrader.
[8] The pharmaceutical composition according to [6] or [7], which is an agent for preventing and/or treating KDM5-related diseases.
[9] The compound or salt according to any one of [1] to [5] for use in the prevention and/or treatment of KDM5-related diseases in which KDM5 inhibitory and KDM5 degrading actions are effective.
[10] The pharmaceutical composition according to any one of [6] to [8] for use in the prevention and/or treatment of KDM5-related diseases in which KDM5 inhibitory and KDM5 degrading actions are effective.
[11] A method for preventing and/or treating a KDM5-related disease in which KDM5 inhibitory and KDM5 degrading actions are effective, the method comprising administering to a patient the compound or salt thereof according to any one of [1] to [5] and [9].
[12] A method for preventing and/or treating a KDM5-related disease in which KDM5 inhibitory and KDM5 degrading actions are effective, the method comprising administering to a patient the pharmaceutical composition according to any one of [6] to [8].
[13] Use of the compound or salt thereof according to any one of [1] to [5] and [9] for production of a pharmaceutical composition for preventing and/or treating a KDM5-related disease in which the KDM5 inhibitory and the KDM5 degrading actions are effective.
[14] An agent for preventing and/or treating KDM5-related diseases, comprising as an active ingredient a compound or a salt thereof according to any one of claims [1] to [5] and [9].

### Advantageous Effects of Invention

According to the present disclosure, it is possible to provide compounds or salts thereof that produce remarkable pharmacological effects, such as anti-cancer effects, in cells and animal studies.

### BRIEF DESCRIPTION OF DRAWINGS

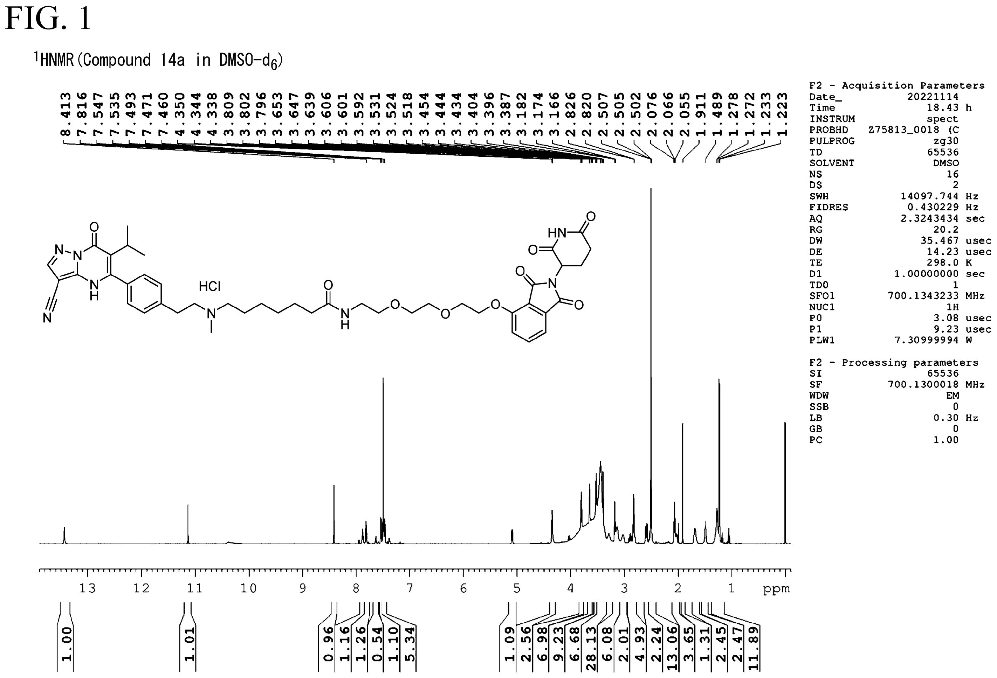

[FIG. 1] FIG. 1 is a diagram showing the ¹H-NMR spectrum of compound 14a.

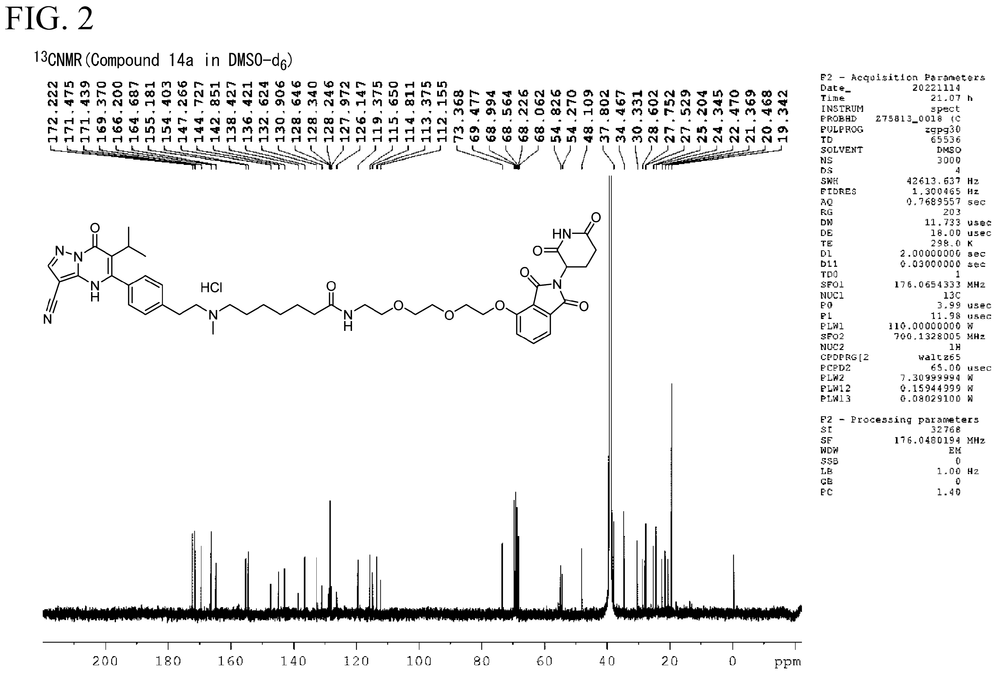

[FIG. 2] FIG. 2 is a diagram showing the ¹³C-NMR spectrum of compound 14a.

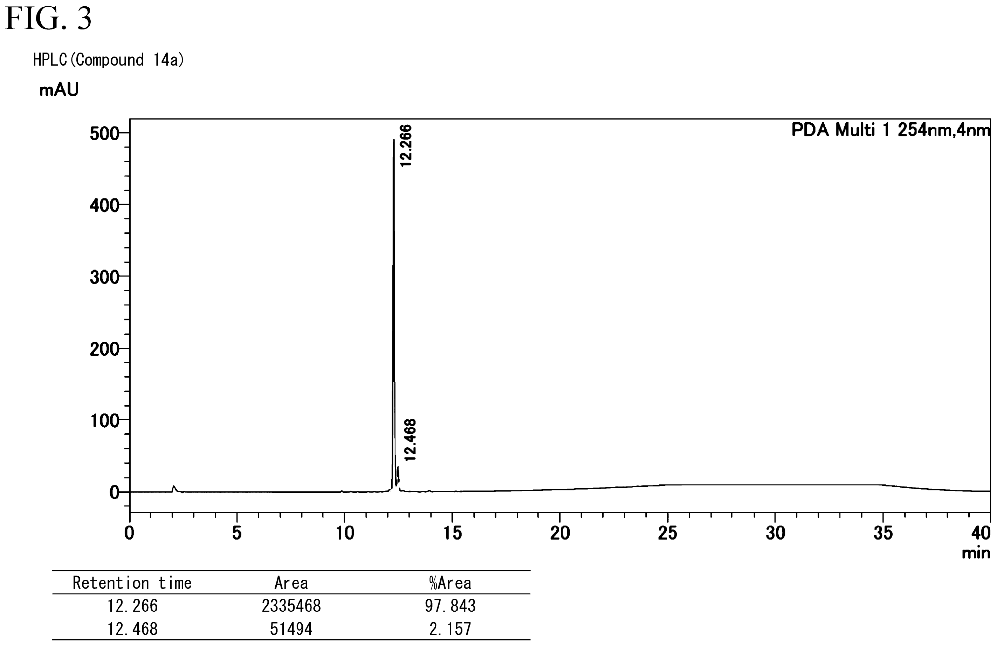

[FIG. 3] FIG. 3 is a diagram showing the HPLC UV absorption spectrum of compound 14a.

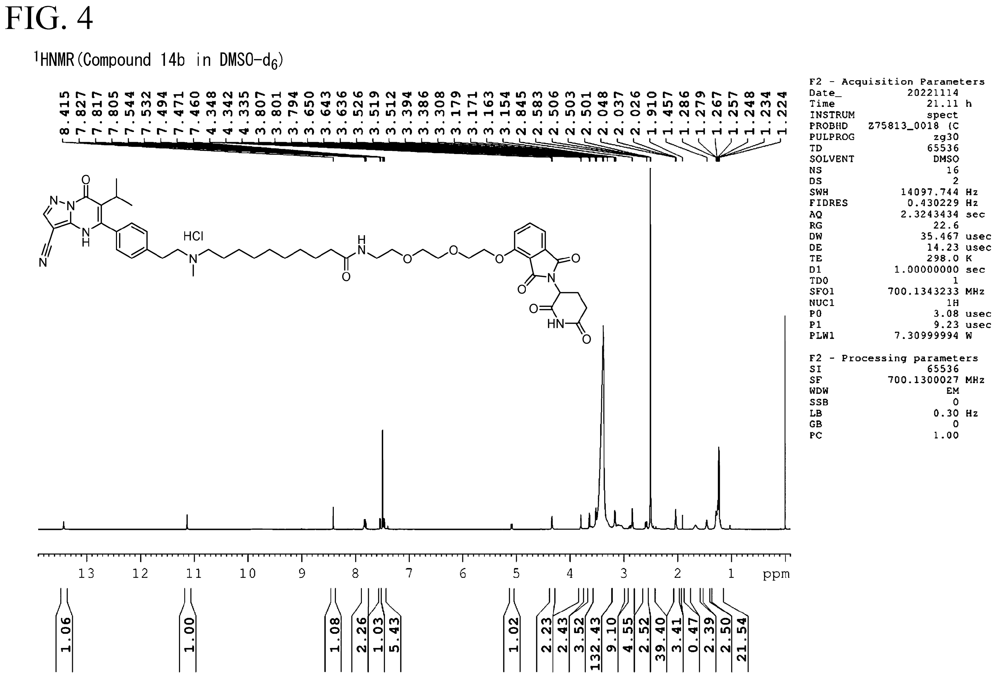

[FIG. 4] FIG. 4 is a diagram showing the ¹H-NMR spectrum of compound 14b.

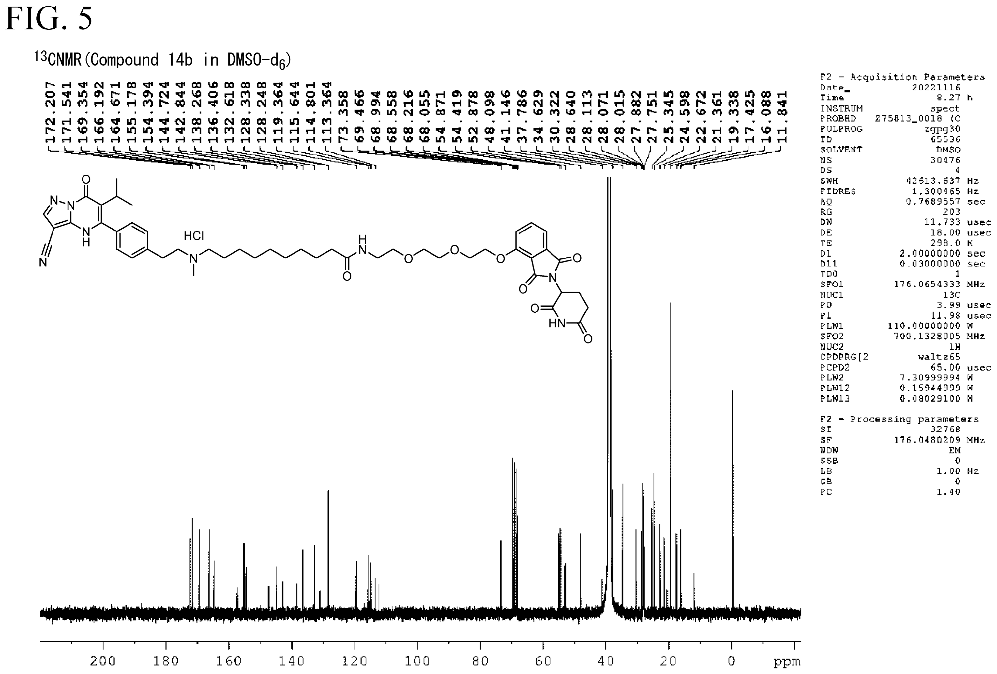

[FIG. 5] FIG. 5 is a diagram showing the ¹³C-NMR spectrum of compound 14b.

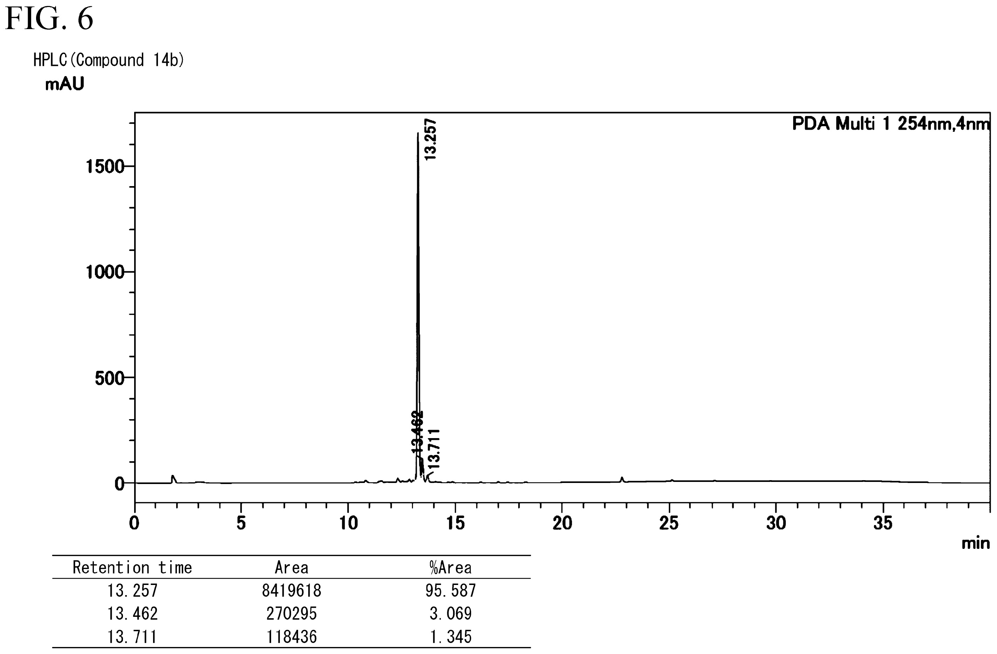

[FIG. 6] FIG. 6 is a diagram showing the HPLC UV absorption spectrum of compound 14b.

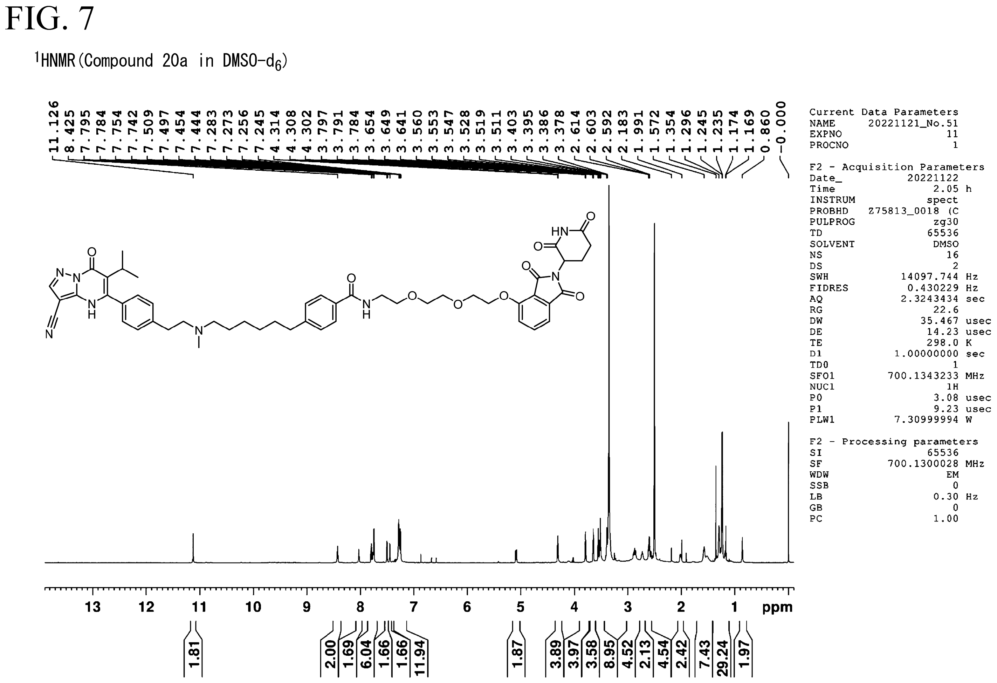

[FIG. 7] FIG. 7 is a diagram showing the ¹H-NMR spectrum of compound 20a.

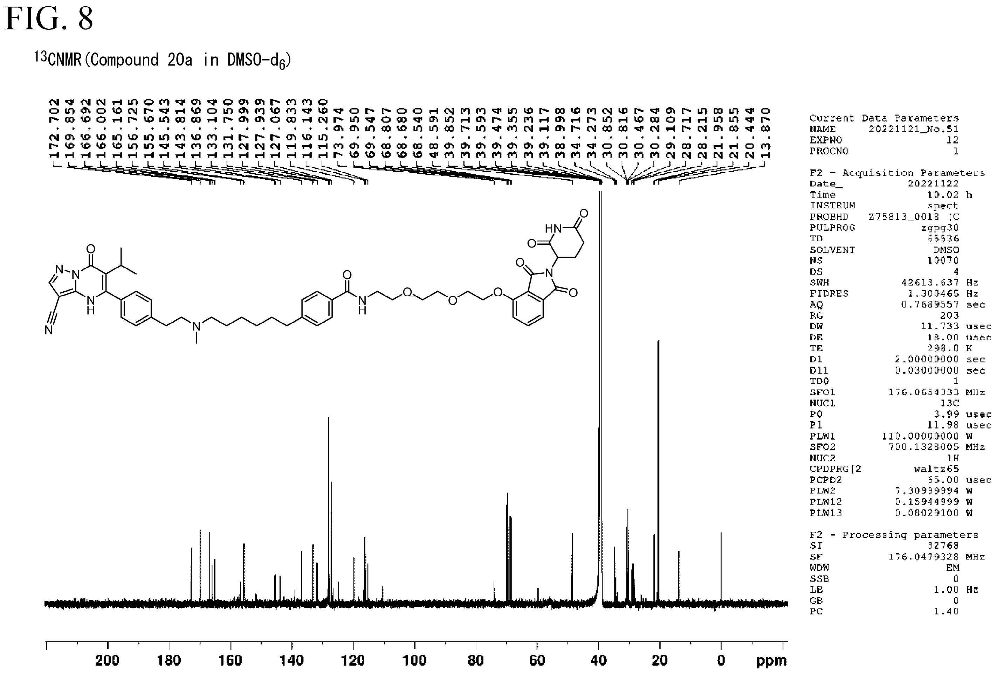

[FIG. 8] FIG. 8 is a diagram showing the ¹³C-NMR spectrum of compound 20a.

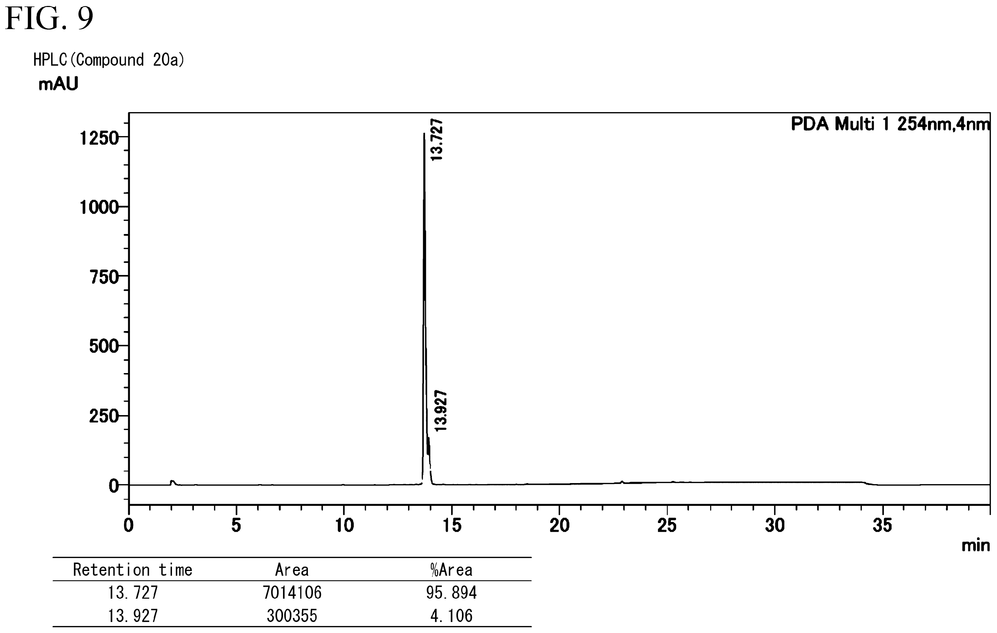

[FIG. 9] FIG. 9 is a diagram showing the HPLC UV absorption spectrum of compound 20a.

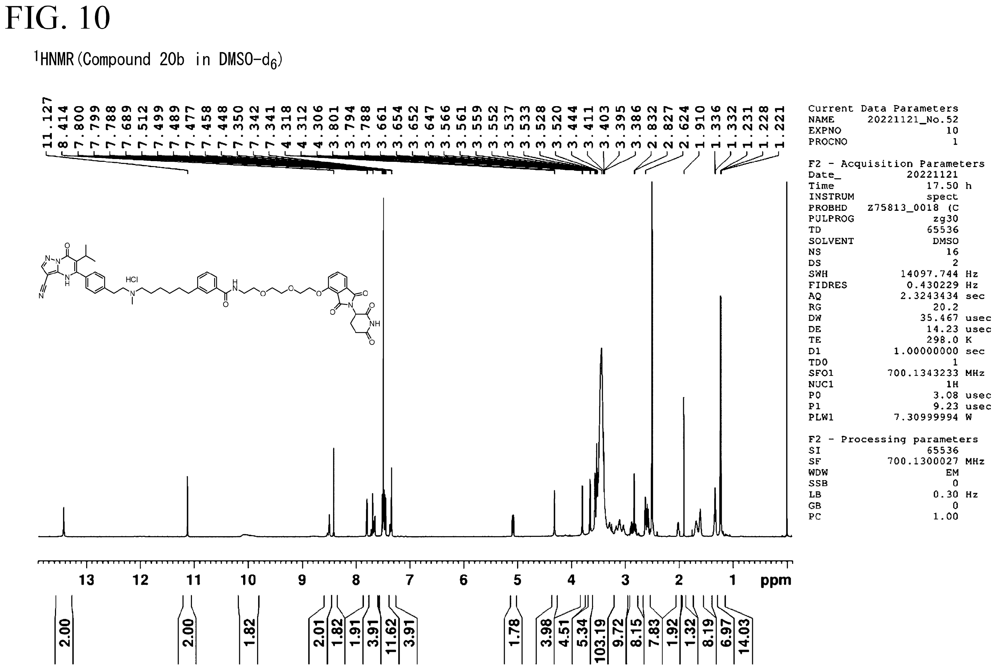

[FIG. 10] FIG. 10 is a diagram showing the ¹H-NMR spectrum of compound 20b.

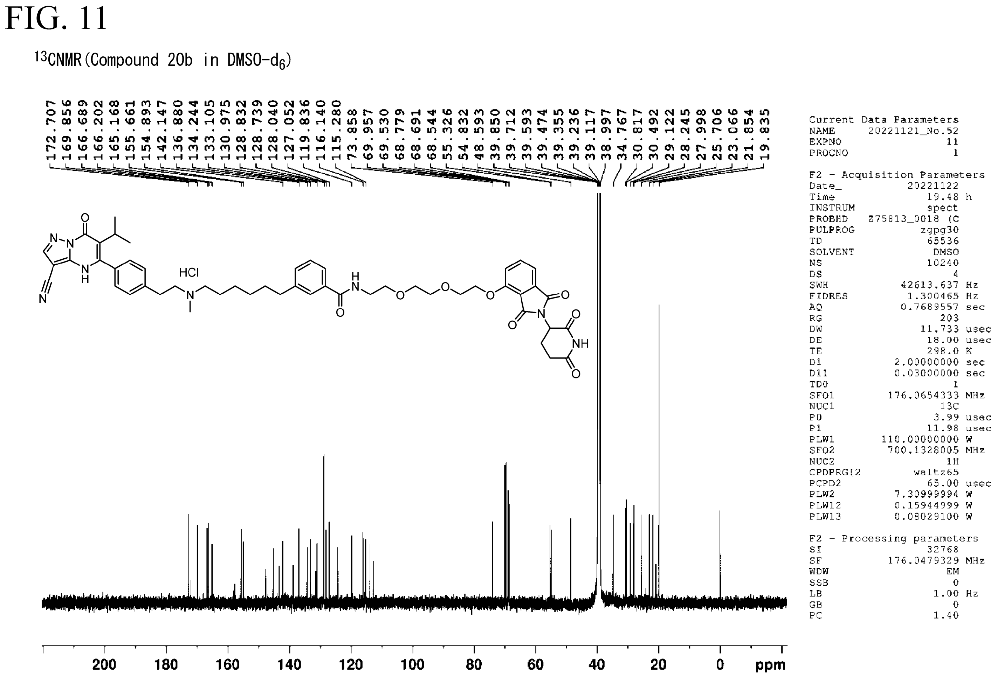

[FIG. 11] FIG. 11 is a diagram showing the ¹³C-NMR spectrum of compound 20b.

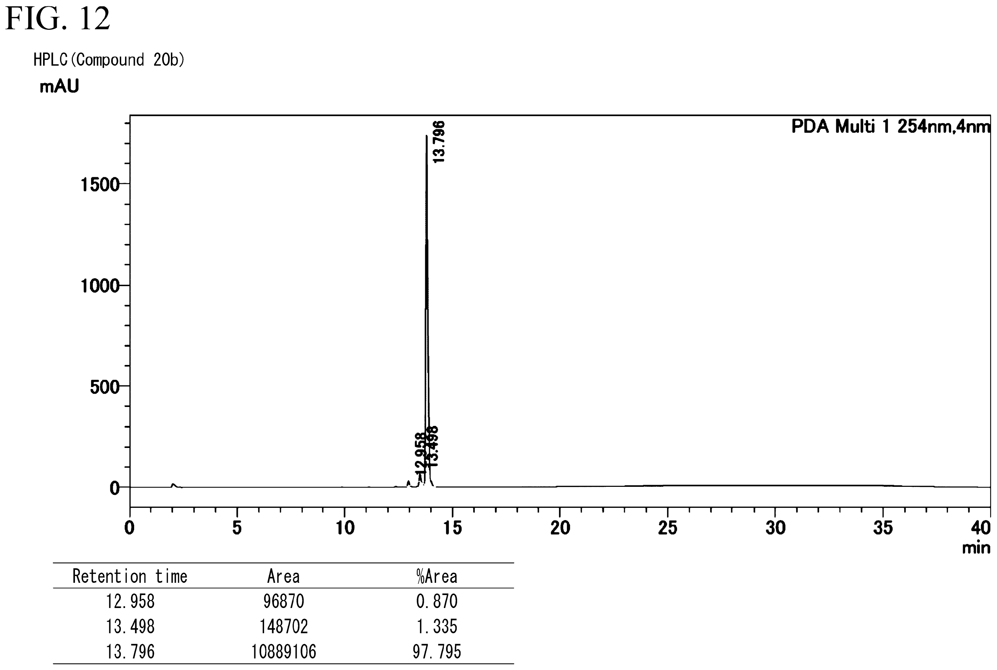

[FIG. 12] FIG. 12 is a diagram showing the HPLC UV absorption spectrum of compound 20b.

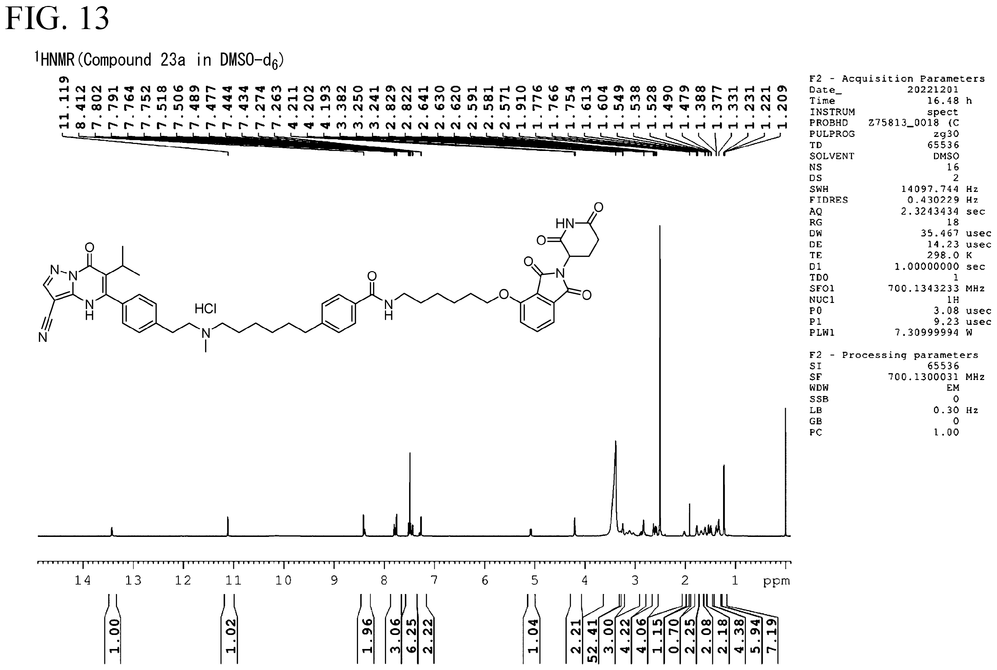

[FIG. 13] FIG. 13 is a diagram showing the ¹H-NMR spectrum of compound 23a.

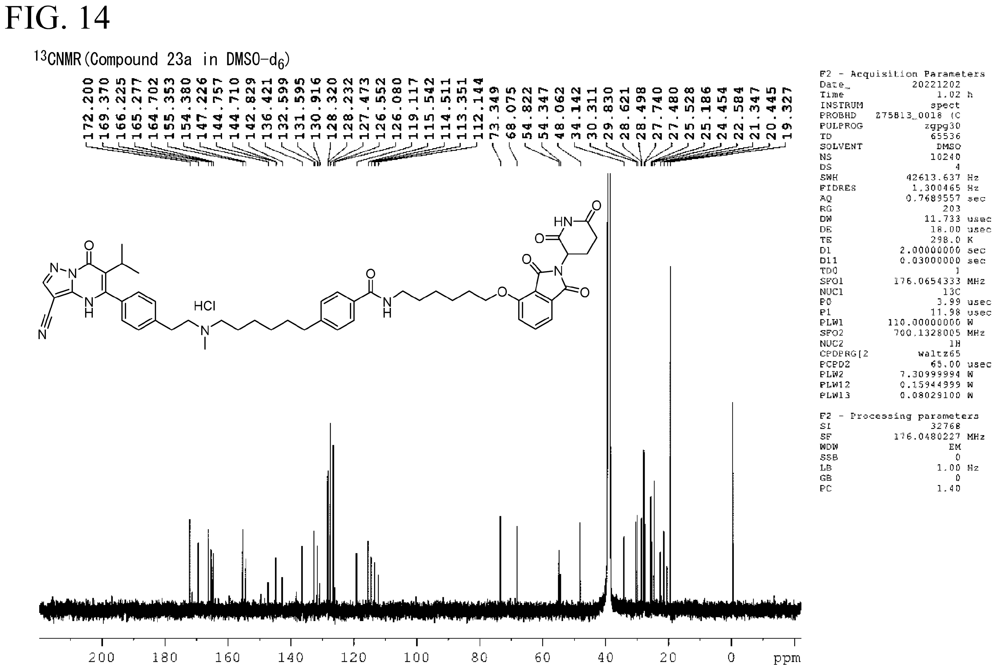

[FIG. 14] FIG. 14 is a diagram showing the ¹³C-NMR spectrum of compound 23a.

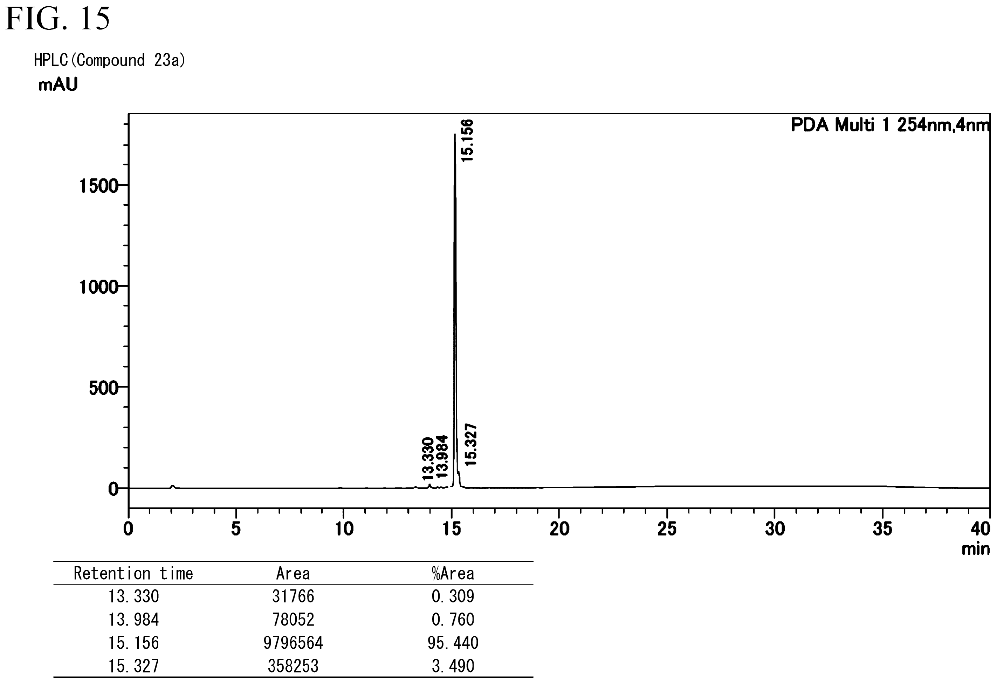

[FIG. 15] FIG. 15 is a diagram showing the HPLC UV absorption spectrum of compound 23a.

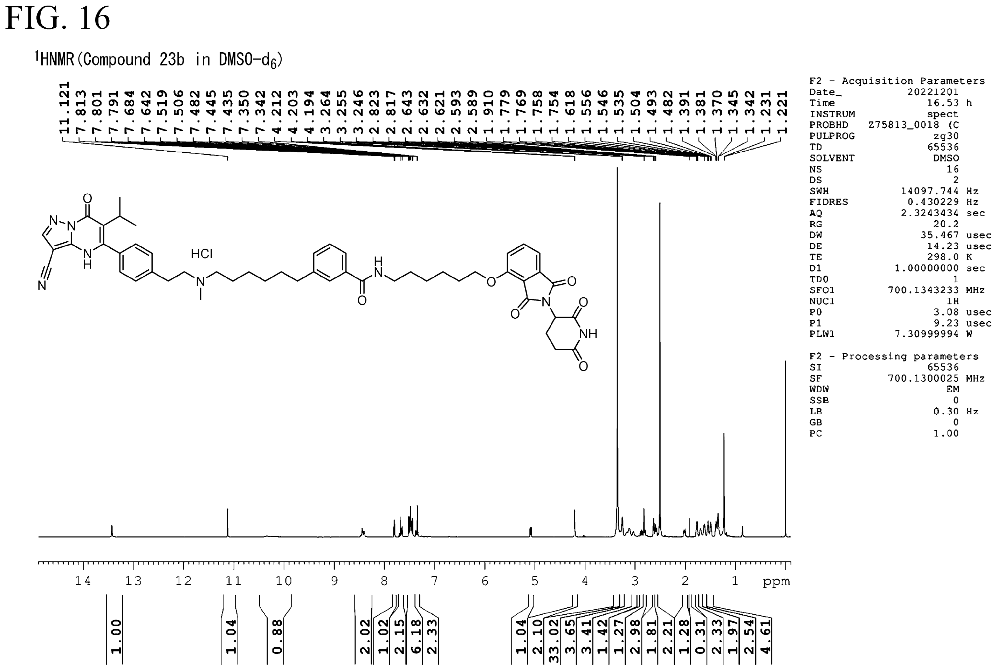

[FIG. 16] FIG. 16 is a diagram showing the ¹H-NMR spectrum of compound 23b.

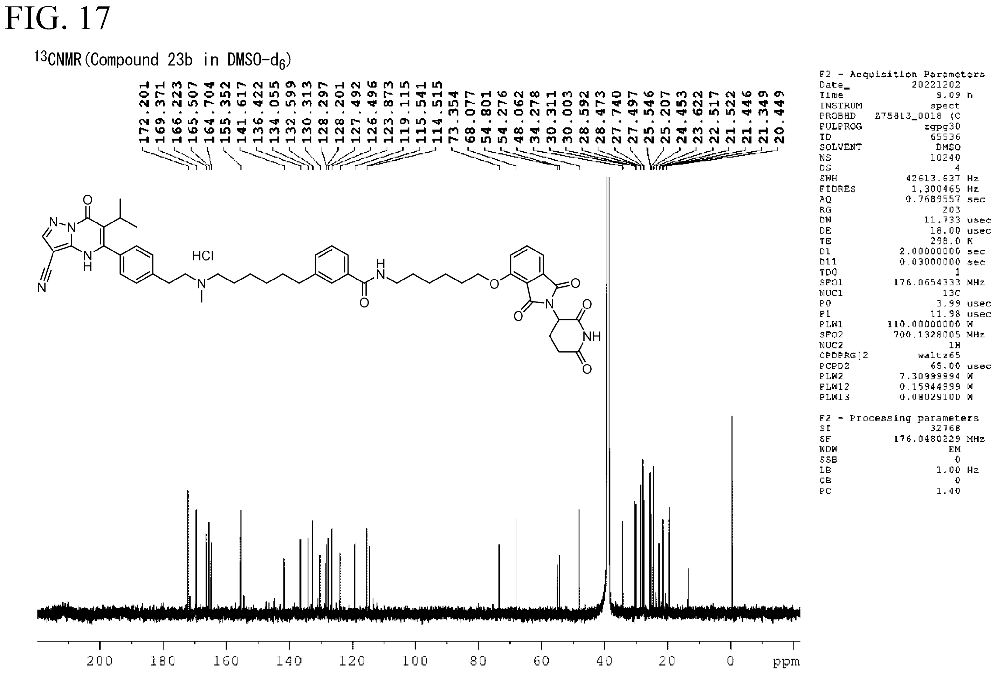

[FIG. 17] FIG. 17 is a diagram showing the ¹³C-NMR spectrum of compound 23b.

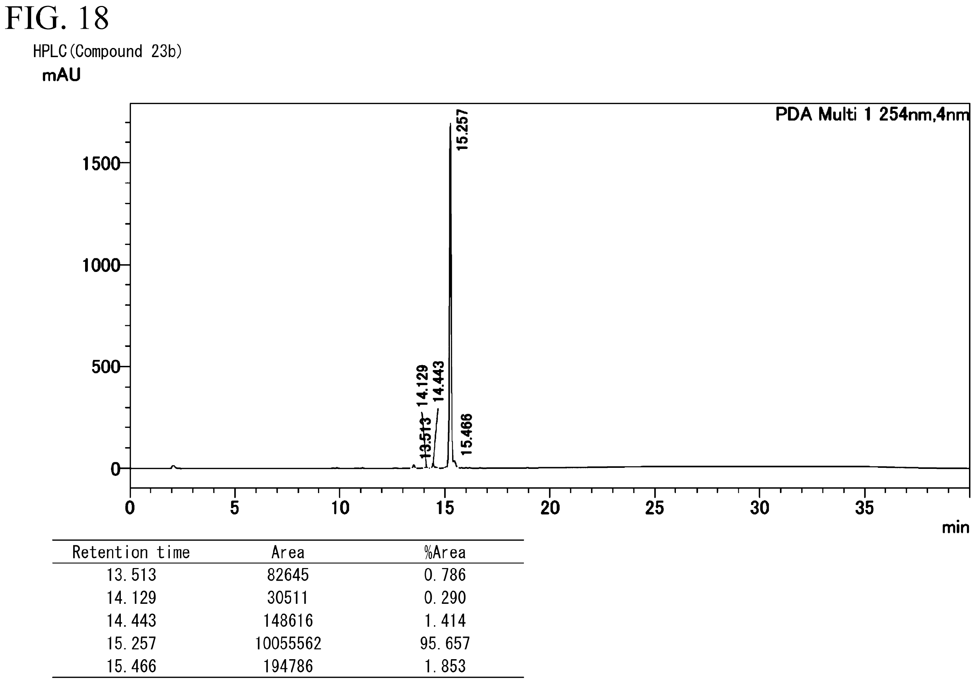

[FIG. 18] FIG. 18 is a diagram showing the HPLC UV absorption spectrum of compound 23b.
[FIG. 19] FIG. 19 (A) shows the structures of KDM5 inhibitor 24 and vorinostat (25), and FIG. 19 (B) shows the effect of single treatment with compounds 24 and 25 and the effect of co-treatment with compounds 24 and 25 on N2a differentiation after 24-hour and 48-hour treatment.
[FIG. 20] FIG. 20 shows representative images of N2a cells treated with single treatment of compounds 24 and 25, and co-treatment of compounds 24 and 25, for 24 and 48 hours. Scale bar is 100 µm.
[FIG. 21] FIG. 21 (A) shows the design of KDM5 PROTACs, FIG. 21 (B) shows the effects of 14a, 14b, 20a, 20b, 23a, and 23b at 0.2 µM on N2a differentiation after 48-hour treatment, FIG. 21 (C) shows the dose-dependent N2a cell-neurite outgrowth activities of compounds 20b, 23b, and 26 after 48-hour treatment, and FIG. 21 (D) shows the time-dependent N2a cell-neurite outgrowth activities of 20b and 23b at 0.02 µM. Bar graphs represent mean values ± SD from three independent experiments.
[FIG. 22] FIG. 22 shows representative images of N2a cells treated with compounds 14a, 14b, 20a, 20b, 23a, and 23b for 48 hours. Scale bar is 100 µm.
[FIG. 23] FIG. 23 shows representative images of N2a cells treated with compounds 20b, 23b, and 26 for 48 hours. Scale bar is 100 µm.
[FIG. 24] FIG. 24 shows representative images of N2a cells treated with compounds 20b and 23b for 24, 48, and 72 hours. Scale bar is 100 µm.
[FIG. 25] FIG. 25 is a diagram showing Western blot-detection of KDM5A levels in N2a cells. (A) after 24-hour treatment with compounds 20b and 23b, (B) after 24-hour treatment with compound 23b, and after 24-hour co-treatment with compounds 24 and 26, (C) after 24-hour co-treatment with compound 20b and proteasome inhibitor MG-132, and (D) after 24-hour co-treatment with proteasome inhibitor MG-132 and compound 23b.
[FIG. 26] FIG. 26 is a diagram showing Western blot-detection of H3K4me3 levels and H3K27Ac levels in N2a cells after 24-hour treatment with compounds 20b and 23b.
[FIG. 27] FIG. 27 is a diagram showing the KDM5A inhibitory activity and HDAC1 inhibitory activity of compounds 20b and 23b.

### DESCRIPTION OF EMBODIMENTS

The present disclosure is described in more detail below.

In addition to the compounds represented by formula (I) and salts thereof, the present disclosure includes their N-oxides, solvates, isomers, mixtures of multiple isomers, single crystal forms, mixtures of multiple crystal forms, co-crystals, and isotope-labeled compounds. They are hereinafter also referred to collectively as "the present compounds".

Unless otherwise specified, "alkyl group" includes linear, branched, and cyclic monovalent saturated hydrocarbon groups. The same applies to alkyl groups in alkoxy groups.

Unless otherwise specified, "alkylene group" includes linear, branched, and cyclic divalent saturated hydrocarbon groups.

Unless otherwise specified, "alkenylene group" includes linear, branched, and cyclic divalent unsaturated hydrocarbon groups.

"Halogen atom" includes fluorine atom, chlorine atom, bromine atom and iodine atom.

The term "substituted or unsubstituted" includes the case where a hydrogen atom (-H) is substituted with a monovalent substituent and the case where a methylene group (-CH₂-) or a methine group (>CH-, or =CH-) is replaced with a divalent group.

"Monovalent substituents" include alkyl groups, alkoxy groups, halogen atoms, alkyl halides, hydroxy groups, carboxy groups, amino groups, nitro groups, etc.

As alkyl groups as substituents, alkyl groups with 1 to 5 carbon atoms are preferred, and methyl, ethyl, propyl, n-butyl, and tert-butyl groups are most preferred.

As alkoxy groups as substituents, alkoxy groups with 1 to 5 carbon atoms are preferred, methoxy, ethoxy, n-propoxy, iso-propoxy, n-butoxy, and tert-butoxy groups are more preferred, and methoxy and ethoxy groups are most preferred.

As halogen atoms as substituents, fluorine and iodine atoms are preferred.

Alkyl halide groups as substituents include alkyl groups having 1 to 5 carbon atoms, such as methyl, ethyl, propyl, n-butyl and tert-butyl groups, wherein some or all of the hydrogen atoms are substituted with halogen atoms.

"Divalent substituent" includes -O-, -C(=O)-O-, -O-C(=O)-, -C(=O)-, -O-C(=O)-O-, - C(=O)-NH-, -C(=O)-NH-C(=O)-, -N=, -NH-, -NH-C(=NH)- (wherein H may be substituted with a substituent such as a hydrocarbon group, acyl group, alkoxyalkyl group, etc.), -S-, -S(=O)₂-, -S(=O)₂-O-, a group represented by the general formula -Y²¹-O-Y²²-, -Y²¹-O-, -Y²¹-C(=O)-O-, -C(=O)-O-Y²¹-, -[Y²¹-C(=O)-O]ₘ"- Y²²-, -Y²¹-O-C(=O)-Y²²- or -Y²¹-S(=O)₂-O-Y²²- [wherein Y²¹ and Y²² are each independently a substituted or unsubstituted divalent hydrocarbon group, O is an oxygen atom, and m" is an integer of 0 to 3], and the like.

### <<The present compound>>

In the compounds of the present disclosure or salts thereof, said compounds are compounds represented by Formula (I).

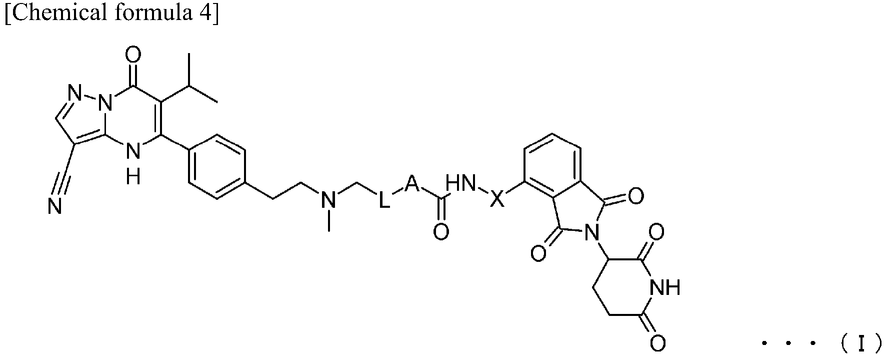

In Formula (I), A is a single bond or a substituted or unsubstituted divalent aromatic hydrocarbon group; L is a substituted or unsubstituted linear or branched alkylene group having 1 to 13 carbon atoms; and X is -(CH₂CH₂O)ₛ-, -(CH₂CH₂CH₂O)ₛ-, -(CH(CH₃)CH₂O)ₛ-, or -(CH₂)ₜO-, wherein s is a number from 1 to 8 and t is a number from 2 to 10.

Said A is: preferably a single bond, a phenylene group, a naphthylene group, or the like; more preferably a single bond, 1,2-phenylene group, 1,3-phenylene group (m-phenylene group), 1,4-phenylene group (p-phenylene group), 1,2-naphthylene group, 1,4-naphthylene group, 1,5-naphthylene group, 2,3-naphthylene group, 2,6-naphthylene group, or the like; and still more preferably a single bond, 1,3-phenylene group (m-phenylene group), and 1,4-phenylene group (p-phenylene group).

Said L is preferably a linear alkylene group having 3 to 10 carbon atoms, more preferably a linear alkylene group having 4 to 9 carbon atoms, and still more preferably a linear alkylene group having 5 to 8 carbon atoms.

Said X is preferably a group represented by -(CH₂CH₂O)ₛ-, -(CH₂CH₂CH₂O)ₛ-, - (CH(CH₃)CH₂O)ₛ-, or -(CH₂)ₜO-; more preferably a group represented by -(CH₂CH₂O)ₛ-, - (CH₂CH₂CH₂O)ₛ-, or -(CH₂)ₜO-; and still more preferably a group represented by -(CH₂CH₂O)ₛ- or - (CH₂)ₜO-.

Said s is preferably a number from 1 to 5, more preferably 2 to 4, still more preferably 2 or 3, and particularly preferably 3.

Said t is preferably a number from 2 to 10, more preferably 3 to 8, still more preferably 4 to 7, and particularly preferably 6.

It is preferable that in said formula (I), A is a single bond or phenylene group, L is a linear alkylene group having 3 to 10 carbon atoms, X is a group represented by -(CH₂CH₂O)ₛ-, - (CH₂CH₂CH₂O)ₛ-, -(CH(CH₃)CH₂O)ₛ-, or -(CH₂)ₜO-, s is a number from 1 to 5, and t is a number from 2 to 10.

It is more preferable that in said formula (I), A is a single bond, p-phenylene group, or m-phenylene group, L is a linear alkylene group having 3 to 10 carbon atoms, X is a group represented by -(CH₂CH₂O)ₛ-, -(CH₂CH₂CH₂O)ₛ-, or -(CH₂)ₜO-, s is a number from 1 to 5, and t is a number from 2 to 10.

It is still more preferable that said compound is a compound represented by the following formula (I-1), a compound represented by the following formula (I-2), or a compound represented by the following formula (I-3).

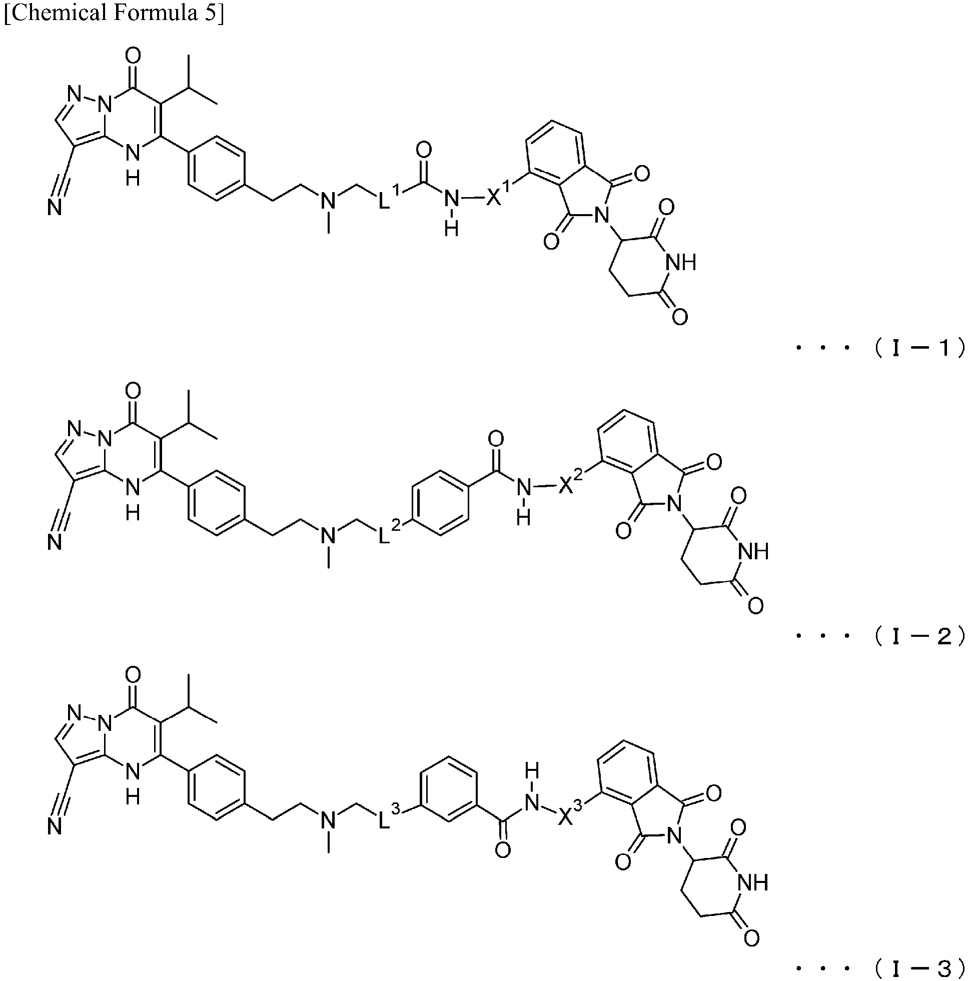

In formula (I-1), L¹ is a linear alkylene group having 3 to 10 carbon atoms, X¹ is a group represented by -(CH₂CH₂O)ₛ-, and s is a number from 1 to 5; in formula (I-2), L² is a linear alkylene group having 3 to 10 carbon atoms, X² is a group represented by -(CH₂CH₂O)ₛ- or -(CH₂)ₜO-, s is a number from 1 to 5, and t is a number from 2 to 10; and in formula (I-3), L³ is a linear alkylene group having 3 to 10 carbon atoms, X³ is a group represented by -(CH₂CH₂O)ₛ- or -(CH₂)ₜO-, s is a number from 1 to 5, and t is a number from 2 to 10.

It is particularly preferable that said compound is at least one selected from the group consisting of: a compound of said formula (I-1), wherein L¹ is a linear alkylene group having 5 carbon atoms, X¹ is a group represented by -(CH₂CH₂O)ₛ-, and s is 3 (14a); a compound of said formula (I-1), wherein L¹ is a linear alkylene group having 8 carbon atoms, X¹ is a group represented by - (CH₂CH₂O)ₛ-, and s is 3 (14b); a compound of said formula (I-2), wherein L² is a linear alkylene group having 5 carbon atoms, X² is a group represented by -(CH₂CH₂O)ₛ-, and s is 3 (20a); a compound of said formula (I-2), wherein L² is a linear alkylene group having 5 carbon atoms, X² is a group represented by -(CH₂)ₜO-, and t is 6 (23a); a compound of said formula (I-3), wherein L³ is a linear alkylene group having 5 carbon atoms, X³ is a group represented by -(CH₂CH₂O)ₛ-, and s is 3 (20b); and a compound of said formula (I-3), wherein L³ is a linear alkylene group having 5 carbon atoms, X³ is a group represented by -(CH₂)ₜO-, and t is 6 (23b).

Salts of said compounds include all pharmacologically acceptable salts. All pharmacologically acceptable salts are preferably low-toxicity water-soluble salts. Examples of suitable salts include acid addition salts (e.g., inorganic acid salts [e.g., hydrochloride, hydrobromide, hydroiodide, sulfate, phosphate, nitrate, etc.], organic acid salts [e.g., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, etc.], salts with acidic natural amino acids [e.g., aspartate, glutamate, etc.]), and the like.

The present compound may be converted to an N-oxide form or a solvate.

The N-oxide form refers to a form in which the nitrogen atom of the present compound is oxidized. The present compound can be converted to an N-oxide form by a known method.

A solvate represents a solvated form with a pharmaceutically acceptable solvent such as water or ethanol. The present compound can be converted to a solvate by a known method.

The present compound may form a co-crystal with a suitable co-crystal former. The co-crystal is preferably a pharmaceutically acceptable one, formed with a pharmaceutically acceptable co-crystal former. A co-crystal is usually defined as a crystal formed by two or more molecules through intermolecular interactions that are not ionic bonds. A co-crystal may be a complex of a neutral molecule and a salt. A co-crystal can be prepared according to known methods such as melt crystallization, recrystallization from a solvent, or physical grinding of the components.

The present disclosure includes all isomers such as geometric isomers, optical isomers based on asymmetric carbons, stereoisomers, and tautomers that are generated from the structure of the compound, as well as mixtures of the isomers, and is not limited to the description of the formula for the sake of convenience; either one isomer or a mixture may be used. Therefore, although there may be asymmetric carbon atoms in the molecule and optical active forms and racemates may exist, the present disclosure is not limited to these, and all are included. Furthermore, although crystal polymorphs may exist, it is similarly not limited, and may be any single crystal form or a mixture thereof, and may be a hydrate in addition to an anhydride, all of which are included in the scope of the present disclosure.

The present disclosure also includes isotopically labeled compounds of the present compound, which are identical to the present compound except that one or more atoms are replaced by an atom having an atomic mass or mass number different from the atomic mass or mass number usually found in nature. Isotopes that can be incorporated into the present compound include, for example, isotopes of hydrogen, carbon, nitrogen, oxygen, fluorine, phosphorus, sulfur, iodine, and chlorine, and include ²H, ³H, ¹¹C, ¹⁴C, ¹³N, ¹⁵O, ¹⁸F, ³⁵S, ¹²³I, and ¹²⁵I, and the like.

The present compound and its pharmaceutically acceptable derivatives (e.g., salts), including the aforementioned isotopes and/or other isotopes are within the scope of the present disclosure. Isotopically labeled compounds of the present disclosure, for example, compounds into which radioactive isotopes such as ³H and/or ¹⁴C are incorporated, are useful in tissue distribution assays of drug and/or substrate. ³H and ¹⁴C are considered useful because of their ease of preparation and detection. The isotopes ¹¹C and ¹⁸F are considered useful in PET (positron emission tomography), and the isotope ¹²⁵I is considered useful in SPECT (single photon emission computed tomography), and all are useful in brain imaging. Substitution with heavier isotopes such as ²H can afford certain therapeutic advantages resulting from greater metabolic stability, for example, increased *in vivo* half-life or reduced dosage requirements, and hence may be useful under certain circumstances. Isotopically labeled compounds of the present compound can be prepared uniformly by carrying out the procedures disclosed in the following schemes and/or examples, using readily available isotopically labeled reagents in place of non-isotopically labeled reagents.

A prodrug of the present compound means a compound that is converted to the present compound by reaction with enzymes, gastric acid, etc., *in vivo*. A prodrug of the present compound may be: when the present compound has an amino group, a compound in which the amino group is acylated, alkylated, or phosphorylated (e.g., the present compound in which the amino group is converted to eicosanoyl, alanyl, pentylaminocarbonyl, (5-methyl-2-oxo-1,3-dioxolen-4-yl)methoxycarbonyl, tetrahydrofuranyl, pyrrolidinylmethyl, pivaloyloxymethyl, acetoxymethyl, tert-butyl, etc.); when the present compound has a hydroxyl group, a compound in which it is acylated, alkylated, phosphorylated, or converted to a borate (e.g., the present compound, etc., in which the hydroxyl group is converted to acetyl, palmitoyl, propanoyl, pivaloyl, succinyl, fumaryl, alanyl, dimethylaminomethylcarbonyl, etc.) and the like. A prodrug of the present compound may be, those which are converted to the present compound under physiological conditions as disclosed in "Development of Pharmaceuticals," Vol. 7, "Molecular Design," 1990, Hirokawa Shoten Co., pages 163-198. A prodrug of the present compound can be produced by a method known per se. A prodrug of the present compound may, like the present compound, form a salt such as an acid addition salt, or may form a solvate with water or an alcoholic solvent (ethanol, etc.).

### <<Method for producing the present compound>>

The present compound can be produced, for example, by the methods described below shown in Scheme I. In the production methods described below, the starting compounds may be salts. As the salts, for example, those described above as salts of the compound represented by general formula (I) are included.

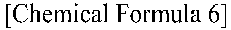

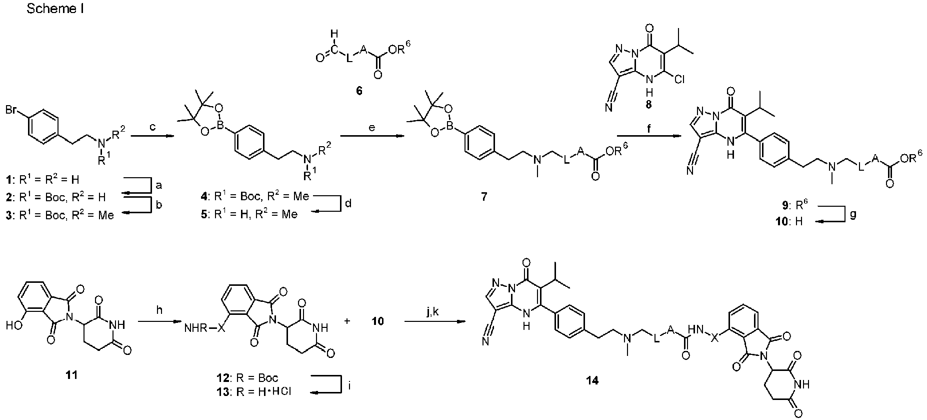

In Scheme I, compounds 1, 6, 8, and 11 are available as commercial products or can be easily synthesized from commercial products by known methods. In the chemical formulas, A, L, and X are the same as A, L, and X described above in the description of the compound represented by formula (I). R⁶ represents an alkyl group having 1 to 6 carbon atoms, and a methyl group, an ethyl group, and the like are preferable.

In step (a), compound 2 can be produced by protecting the primary amino group of compound 1 with a Boc group (t-butoxycarbonyl group).

The protection of a primary amino group with a Boc group is known. For example, it can be carried out in a solvent such as methylene chloride in the presence of a Boc group-introducing agent such as (Boc)₂O and a base such as triethylamine at 0 to 80°C.

In step (b), compound 3 can be produced by replacing the hydrogen atom on the nitrogen atom of compound 2 with a methyl group.

The reaction for replacing a hydrogen atom on a nitrogen atom with a methyl group is known. For example, it can be carried out in a solvent such as N,N-dimethylformamide (hereinafter, also referred to as DMF) in the presence of a methylating agent such as MeI and a base such as NaH at 0 to 80°C.

In step (c), compound 4 can be produced by a cross-coupling reaction between compound 3 and an organoboronic acid compound using a palladium catalyst.

Cross-coupling reactions using a palladium catalyst are known. For example, it can be carried out in a solvent such as dimethyl sulfoxide (hereinafter, also referred to as DMSO) in the presence of compound 3, an organoboronic acid compound such as bis(pinacolato)diboron, a palladium catalyst such as Pd(dppf)Cl₂, and a base such as KOAc at 40 to 190°C.

In step (d), compound 5 can be produced by deprotecting the Boc-protected amino group of compound 4.

The deprotection of a Boc-protected amino group is known. For example, it can be carried out in a solvent such as methylene chloride in the presence of an acid such as trifluoroacetic acid at 0 to 80°C.

In step (e), compound 7 can be produced by reductive amination of the secondary amino group of compound 5 with an organic aldehyde compound 6.

Reductive amination is known. For example, it can be carried out in a solvent such as 1,2-dichloroethane in the presence of a reducing agent such as sodium triacetoxyborohydride at 0 to 80°C.

In step (f), compound 9 can be produced by a cross-coupling reaction between compound 7 and an organic halogen compound 8 using a palladium catalyst.

Cross-coupling reactions using a palladium catalyst are known. For example, it can be carried out in a solvent such as 1,2-dimethoxyethane (hereinafter, also referred to as DME) in the presence of compound 7, compound 8, a palladium catalyst such as Pd(dppf)Cl₂, and a base such as Na₂CO₃ at 40 to 190°C.

In step (g), compound 10 can be produced by hydrolyzing the ester of compound 9 to convert it to a carboxy group.

The hydrolysis reaction to convert an ester to a carboxy group is known. For example, it can be carried out in a solvent such as tetrahydrofuran (hereinafter, also referred to as THF), methanol, and water in the presence of a base such as sodium hydroxide at 0 to 80°C.

In step (h), compound 12 can be produced by an aryl ether synthesis reaction between compound 11 having a phenolic hydroxyl group and an alcohol having a Boc-protected amino group.

The aryl ether synthesis reaction between an organic compound having a phenolic hydroxyl group and an alcohol is known. For example, it can be carried out in a solvent such as THF in the presence of compound 11, said specific alcohol, a Mitsunobu reaction reagent such as di-2-methoxyethyl azodicarboxylate, and an organophosphorus compound such as triphenylphosphine at 0 to 80°C.

Alternatively, in step (h), compound 12 can be produced by an S_{N}2 reaction between compound 11 having a phenolic hydroxyl group and an alkyl halide having a Boc-protected amino group.

The S_{N}2 reaction between compound 11 having a phenolic hydroxyl group and an alkyl halide is known. For example, it can be carried out in a solvent such as DMF in the presence of compound 11, said specific alkyl halide, a base such as KHCO₃, and an iodinating agent such as KI at 0 to 150°C.

In step (i), compound 13 can be produced by deprotecting the Boc-protected amino group of compound 12, and then converting the amino group to a hydrochloride salt.

The deprotection of a Boc-protected amino group is known. For example, it can be carried out in a solvent such as methylene chloride in the presence of an acid such as trifluoroacetic acid at 0 to 80°C.

The reaction to convert an amino group to a salt is known. For example, after deprotection, it can be carried out in ethyl acetate (hereinafter, also referred to as AcOEt) in the presence of an acid such as hydrochloric acid at 0 to 80°C.

In step (j), compound 14 before being converted to a hydrochloride salt can be produced by a condensation reaction between the carboxy group of compound 10 and the secondary amino group of compound 13.

The condensation reaction between a carboxy group and an amino group is known. For example, it can be carried out in DMF in the presence of compound 10, compound 13, a condensation agent such as 1-[bis(dimethylamino)methylene]-1H-1,2,3-triazolo[4,5-b]pyridinium 3-oxide hexafluorophosphate (hereinafter, also referred to as HATU), and a base such as diisopropylethylamine (hereinafter, also referred to as i-Pr₂NEt) at 0 to 80°C.

In step (k), the hydrochloride salt of compound 14 can be produced by converting the tertiary amino group in compound 14 to a hydrochloride salt.

The reaction to convert a tertiary amino group to a salt is known. For example, it can be carried out in AcOEt in the presence of an acid such as hydrochloric acid at 0 to 80°C.

In addition, said compound 6 can also be produced by the methods described below shown in Scheme II.

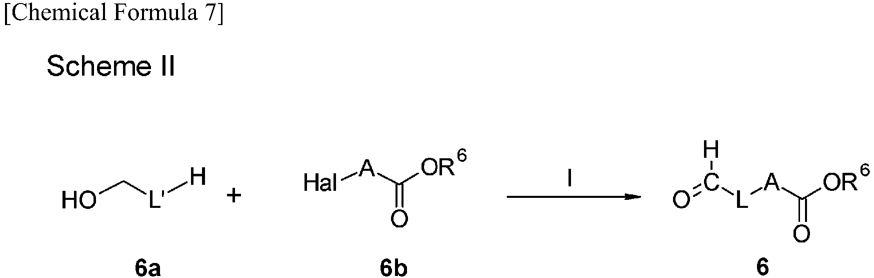

In Scheme II, compounds 6a and 6b are available as commercial products or can be easily synthesized from commercial products by known methods.

In the chemical formula, L is the same as L described above in the description of the compound represented by formula (I).

A is a substituted or unsubstituted divalent aromatic hydrocarbon group, and phenylene group and naphthylene group are preferable; 1,2-phenylene group, 1,3-phenylene group (m-phenylene group), 1,4-phenylene group (p-phenylene group), 1,2-naphthylene group, 1,4-naphthylene group, 1,5-naphthylene group, 2,3-naphthylene group, and 2,6-naphthylene group are more preferable; and 1,3-phenylene group (m-phenylene group) and 1,4-phenylene group (p-phenylene group) are still more preferable.

L' is a substituted or unsubstituted linear or branched alkenylene group having 2 to 13 carbon atoms, preferably a linear alkenylene group having 3 to 10 carbon atoms, more preferably a linear alkenylene group having 4 to 9 carbon atoms, and still more preferably a linear alkenylene group having 5 to 8 carbon atoms.

Hal represents a halogen atom, and a chlorine atom, a bromine atom, and an iodine atom and the like are preferable.

R⁶ represents an alkyl group having 1 to 6 carbon atoms, and a methyl group and an ethyl group and the like are preferable.

In step (l), compound 6 can be produced by a cross-coupling reaction between an unsaturated alcohol 6a and an aryl halide 6b using a palladium catalyst.

Cross-coupling reactions using a palladium catalyst are known. For example, it can be carried out in a solvent such as DMF in the presence of compound 6a, compound 6b, a palladium catalyst such as Pd(OAc)₂, a base such as LiOAc and tetrabutylammonium chloride (hereinafter, also referred to as n-Bu₄NCl), and a reaction accelerator such as LiCl at 40 to 190°C.

### <Use>

The present compound has both KDM5 inhibitory activity and KDM5 degrading activity, and therefore can be used as a KDM5 inhibitor and a KDM5 degrader, and can be used as an agent for preventing and/or treating KDM5-related diseases in mammals, particularly humans.

The present compound can be used as a chemical probe for capturing target proteins of bioactive small molecule compounds. That is, the present compound can be converted into an affinity chromatography probe, a photoaffinity probe, etc., by introducing a labeling group, a linker, etc., into a moiety different from the structural moiety essential for the activity expression of the compound by a method described in J. Mass Spectrom. Soc. Jpn. Vol. 51, No. 5, 2003, p492-498 or WO2007/139149, etc.

Examples of labeling groups, linkers, etc., used for chemical probes include groups shown in the group consisting of the following (1) to (5).
(1) Protein labeling groups such as photoaffinity labeling groups (e.g., benzoyl group, benzophenone group, azide group, carbonyl azide group, diaziridine group, enone group, diazo group, and nitro group, etc.) and chemical affinity groups (e.g., ketone groups in which the alpha carbon atom is substituted with a halogen atom, carbamoyl groups, ester groups, alkylthio groups, Michael acceptors such as α,β-unsaturated ketones and esters, and oxirane groups, etc.),
(2) Cleavable linkers such as -S-S-, -O-Si-O-, monosaccharides (glucose group, galactose group, etc.) or disaccharides (lactose, etc.), and oligopeptide linkers cleavable by enzymatic reaction,
(3) Fishing tag groups such as biotin, 3-(4,4-difluoro-5,7-dimethyl-4H-3a,4a-diaza-4-bora-s-indacene-3-yl)propionyl group,
(4) Detectable markers such as: radioactive labeling groups such as ¹²⁵I, ³²P, ³H, ¹⁴C; fluorescent labeling groups such as fluorescein, rhodamine, dansyl, umbelliferone, 7-nitrofurazanyl, 3-(4,4-difluoro-5,7-dimethyl-4H-3a,4a-diaza-4-bora-s-indacene-3-yl)propionyl group; chemiluminescent groups such as luciferin, luminol; heavy metal ions such as lanthanide metal ions, radium ions, or
(5) Groups for binding to a solid phase carrier such as glass beads, glass beds, microtiter plates, agarose beads, agarose beds, polystyrene beads, polystyrene beds, nylon beads, nylon beds, etc.

A probe prepared by introducing a labeling group, etc., selected from the group consisting of (1) to (5) above into the present compound according to the method described in the above literature, etc., can be used as a chemical probe for identifying labeled proteins useful for searching for new drug targets, etc.

### <<Pharmaceutical Compositions>>

The pharmaceutical composition of the present disclosure (hereinafter, collectively referred to as the present pharmaceutical composition) comprises the present compound.

When the present compound is used for pharmaceutical purposes, it can be used not only as a single agent but also as a combination drug with other drugs for the purpose of, for example, (1) supplementing and/or enhancing the effects of the present compound for the prevention, treatment, and/or improvement of symptoms, (2) improving pharmacokinetics and absorption, reducing the dosage of the present compound, and/or (3) reducing side effects of the present compound.

The pharmaceutical composition of the present disclosure can contain 0.000001 to 99.5% by weight, preferably 0.000001 to 90% by weight, etc., of the present compound with respect to the total mass of the pharmaceutical composition.

In combination with other drugs, the present compound and the other drug may be administered as a combination drug containing both components in a single preparation, or separate preparations may be administered by the same or different administration routes. It is not necessary to administer separate preparations at the same time, and they may be administered sequentially with a time lag. When administered sequentially, the order of administration or the administration method is not particularly limited and can be appropriately adjusted to obtain the expected efficacy.

The dosage of other drugs used in combination with the present compound can be appropriately increased or decreased according to its clinical dosage or similar drugs. The ratio of the present compound to other drugs can be appropriately adjusted in consideration of the subject's age, weight, administration method, administration time, target disease, pathological condition, etc. Usually, other drugs can be combined in a range of 0.01 to 100 parts by weight with respect to 1 part by weight of the present compound. A plurality of other drugs may be used. The other drugs may have a similar mechanism of action as the above-mentioned drugs, in addition to the above-mentioned drugs. The said other drugs include not only known ones but also those to be discovered in the future.

The dosage of the present compound varies depending on age, body weight, pathological condition, therapeutic effect, administration method, administration period, etc. The present compound may be orally administered to an adult in a range of 0.1 mg to 300 mg per dosage once to several times a day, or parenterally administered to an adult in a range of 0.1 mg to 150 mg per dosage once to several times a day, or continuously administered intravenously for 1 to 24 hours a day.

The pharmaceutical composition comprising the present compound can be formulated into various dosage forms. Examples of dosage forms include oral administration agents (e.g., tablets, capsules, granules, powders, oral solutions, syrups, oral jellies, etc.), oral preparations (e.g., oral tablets, oral sprays, oral semi-solid preparations, oral gargles, etc.), injectable preparations (e.g., injections, etc.), preparations for dialysis (e.g., agents for dialysis, etc.), inhalation preparations (e.g., inhalants, etc.), ophthalmic preparations (e.g., eye drops, eye ointments, etc.), otolaryngological preparations (e.g., ear drops, etc.), nasal preparations (e.g., nasal drops, etc.), rectal preparations (e.g., suppositories, rectal semi-solid preparations, enemas, etc.), vaginal preparations (e.g., vaginal tablets, vaginal suppositories, etc.), and dermal preparations (e.g., solid external preparations, liquid external preparations, sprays, ointments, creams, gels, patches, etc.).

When producing an oral solid preparation, excipients, binders, disintegrants, lubricants, coloring agents, etc., may be added to the present compound as necessary, and tablets, granules, powders, and capsules can be produced by a conventional method. Also, tablets, granules, powders, capsules, etc., may be film-coated as necessary.

Examples of excipients include lactose, corn starch, crystalline cellulose, etc., examples of binders include hydroxypropyl cellulose, hydroxypropyl methylcellulose, etc., examples of disintegrants include carboxymethyl cellulose calcium, croscarmellose sodium, etc., examples of lubricants include magnesium stearate, calcium stearate, etc., examples of coloring agents include titanium oxide, etc., and examples of film-coating agents include hydroxypropyl cellulose, hydroxypropyl methylcellulose, methyl cellulose, etc., but are of course not limited to these.

These solid preparations such as tablets, capsules, granules, and powders can usually contain 0.001 to 99.5% by weight, preferably 0.001 to 90% by weight, etc., of the present compound with respect to the total mass of said solid preparation.

When producing an injection (for intravenous administration, intramuscular administration, subcutaneous administration, intraperitoneal administration, etc.), a pH adjuster, a buffer, a suspending agent, a solubilizing agent, an antioxidant, a preservative (antiseptic), an isotonic agent, etc., are added to the present compound as necessary, and an injection can be produced by a conventional method. It may also be freeze-dried to produce a lyophilized preparation to be dissolved at the time of use.

Examples of pH adjusters and buffers include organic acids or inorganic acids and/or their salts, etc., examples of suspending agents include methyl cellulose, polysorbate 80, sodium carboxymethyl cellulose, etc., examples of solubilizing agents include polysorbate 80, polyoxyethylene sorbitan monolaurate, etc., examples of antioxidants include α-tocopherol, etc., examples of preservatives include methyl parahydroxybenzoate, ethyl parahydroxybenzoate, etc., and examples of isotonic agents include glucose, sodium chloride, mannitol, etc., but are of course not limited to these.

These injections can usually contain 0.000001 to 99.5% by weight, preferably 0.000001 to 90% by weight, etc., of the present compound with respect to the total mass of the injection.

When producing an external preparation, a base raw material is added to the present compound, and as necessary, the above-mentioned preservatives, stabilizers, pH adjusters, antioxidants, coloring agents, etc., are added, and transdermal absorption preparations (ointments, patches, etc.), eye drops, nasal drops, suppositories, etc., can be produced by a conventional method.

As the base raw materials to be used, various raw materials usually used in pharmaceuticals, quasi-drugs, cosmetics, etc., can be used. Specifically, raw materials such as animal and vegetable oils, mineral oils, ester oils, waxes, emulsifiers, higher alcohols, fatty acids, silicone oils, surfactants, phospholipids, alcohols, polyhydric alcohols, water-soluble polymers, clay minerals, and purified water can be mentioned.

These external preparations can usually contain 0.000001 to 99.5% by weight, preferably 0.000001 to 90% by weight, etc., of the present compound with respect to the total mass of the external preparation.

The dosage of the present compound varies depending on the degree of symptoms, age, sex, body weight, administration form/salt type, specific type of disease, etc., but usually, it is about 30 µg to 10 g for oral administration, preferably 100 µg to 5 g, more preferably 100 µg to 1 g, and for injection, about 30 µg to 1 g, preferably 100 µg to 500 mg, more preferably 100 µg to 300 mg, per day for adults, administered once or in several divided doses.

### <Use>

The present compound and the present pharmaceutical composition have both KDM5 inhibitory activity and KDM5 degrading activity, and therefore can be used as a KDM5 inhibitor and a KDM5 degrader, and can be used as an agent for preventing and/or treating KDM5-related diseases (in mammals, particularly humans).

Examples of such diseases include hyperproliferative disorders, cancer, stroke, diabetes, hepatomegaly, cardiovascular disease, multiple sclerosis, Huntington's disease, Alzheimer's disease, cystic fibrosis, viral diseases, autoimmune diseases, atherosclerosis, restenosis, psoriasis, rheumatoid arthritis, inflammatory bowel disease, asthma, allergic diseases, inflammation, neuropathy, hormone-related diseases, symptoms associated with organ transplantation, immunodeficiency diseases, destructive bone disorders, proliferative diseases, infectious diseases, symptoms associated with cell death, thrombin-induced platelet aggregation, liver diseases, pathological immune conditions involving T-cell activity, central nervous system disorders, myeloproliferative disorders, Parkinson's disease, Lewy body disease, frontotemporal dementia, mild cognitive impairment, dementia, cerebrovascular disease, schizophrenia, depression, anxiety disorders, bipolar disorder, autism spectrum disorder, attention deficit/hyperactivity disorder, learning disabilities, motor disorders, obsessive-compulsive disorder, personality disorders, sleep disorders, delirium, amyotrophic lateral sclerosis, developmental disorders, intellectual disabilities, post-traumatic stress disorder, hepatitis, and the like.

In particular, the present compound and the present pharmaceutical composition are useful for the prevention and/or treatment of cancer, Huntington's disease, Alzheimer's disease, Parkinson's disease, Lewy body disease, frontotemporal dementia, mild cognitive impairment, dementia, cerebrovascular disease, schizophrenia, depression, anxiety disorders, bipolar disorder, autism spectrum disorder, attention deficit/hyperactivity disorder, learning disabilities, motor disorders, obsessive-compulsive disorder, personality disorders, sleep disorders, delirium, amyotrophic lateral sclerosis, developmental disorders, intellectual disabilities, post-traumatic stress disorder, or hepatitis. The present compound or the present pharmaceutical composition is particularly suitable for the prevention and/or treatment of cancer and Alzheimer's disease.

When the present pharmaceutical composition is used for the prevention and/or treatment of the above diseases as a single agent or in combination with other drugs, the active ingredient, the present compound, is usually formulated with pharmaceutically acceptable carriers such as various additives or solvents, and the obtained preparation is administered systemically or locally, orally or parenterally. Here, a pharmaceutically acceptable carrier means a substance other than the active ingredient that is commonly used in pharmaceutical preparations. The pharmaceutically acceptable carrier preferably exhibits no pharmacological activity at the dosage of the preparation, is harmless, and does not interfere with the therapeutic effect of the active ingredient. The pharmaceutically acceptable carrier may be used to enhance the usefulness of the active ingredient and the preparation, facilitate formulation, stabilize quality, or improve usability. Specifically, substances described in the "Dictionary of Pharmaceutical Additives" 2000, Yakuji Nippo, Ltd. (IPEC JAPAN version) can be appropriately selected as needed.

### <<Method for producing pharmaceutical composition>>

A pharmaceutical composition comprising the present compound can be produced by mixing the present compound with other drugs, carriers, excipients, and other additives as necessary.

### Examples

The following Examples are provided to specifically illustrate the present disclosure, and are not intended to limit the scope of the disclosure in any way.

The chemical reagents and solvents used in this study were commercial products of the highest available purity. Reagents and solvents were purchased from Sigma-Aldrich, FUJIFILM Wako Pure Chemical Corporation, TCI Tokyo Chemical Industry Co., Ltd., Nacalai Tesque, Inc., and Kanto Chemical Co., Inc., and used without purification.

All air- and moisture-sensitive reactions were performed under an argon (Ar) atmosphere in dried glassware. NMR spectra were recorded on a JEOL ECS400 spectrometer operating at 400 MHz (¹H), and Bruker AVANCE III 700 spectrometer operating at 700 MHz (¹H) or 175 MHz (¹³C). ¹H-NMR and ¹³C-NMR chemical shift values are reported as δ (ppm) relative to the solvent peak or tetramethylsilane (TMS) (DMSOd6: ¹H-NMR 2.50, ¹³C-NMR 39.52), and coupling constants are given in Hz.

The purity of all tested compounds was >95% as determined via HPLC using a Shimadzu UFLC (SPD-M20A UV detector, DGU-20A3R degassing unit, LC-20AD solvent delivery unit, and CBM-20A system) and COSMOSIL packed column (5C18-AR-II, 4.6 ID x 150 mm, Nacalai Tesque, Inc.) at a flow rate of 1 mL/min, with UV detection (λ = 254 nm).

### HPLC conditions:

Eluent A: H₂O containing 0.1% TFA; eluent B: acetonitrile containing 0.1% TFA.
Eluent B: 0 to 20 min, 10-90%; 20 to 30 min, 90%; 30 to 40 min, 90-10%. Positive/negative LRMS ion mass spectra were recorded on Bruker HCT-Plus. High-resolution mass spectra (HRMS) were recorded on a LTQ Orbitrap XL (THERMO) or Shimadzu LCMS-IT-TOF mass spectrometer.

### Abbreviations

AcOEt: ethyl acetate;
Boc₂O = di-t-butyl dicarbonate;
n-Bu₄NCl: tetrabutylammonium chloride;
DME: 1,2-dimethoxyethane;
DMEAD = di-2-methoxyethyl azodicarboxylate;
DMF = N,N-dimethylformamide;
DMSO = dimethyl sulfoxide;
dppf = 1,1'-ferrocene bis(diphenylphosphine);
HATU = 1-[bis(dimethylamino)methylene]-1H-1,2,3-triazolo[4,5-b]pyridinium 3- oxide hexafluorophosphate;
MeCN: acetonitrile;
MeI: methyl iodide;
MeOH: methanol;
i-Pr₂NEt: diisopropylethylamine;
TFA = trifluoroacetic acid;
THF = tetrahydrofuran.

### (1) Synthesis of compounds

### <<Example 1: Synthesis of 7-{[4-(3-cyano-6-isopropyl-7-oxo-4,7-dihydropyrazolo[1,5-a]pyrimidin-5-yl)phenethyl](methyl)amino}-N-{2-[2-(2,6-dioxopiperidin-3-yl)-1,3-dioxoindolin-4-yl]oxy}ethoxy}ethyl} heptanamide hydrochloride (14a•HCl) >>

Compound **14a** was synthesized according to the following reaction scheme.

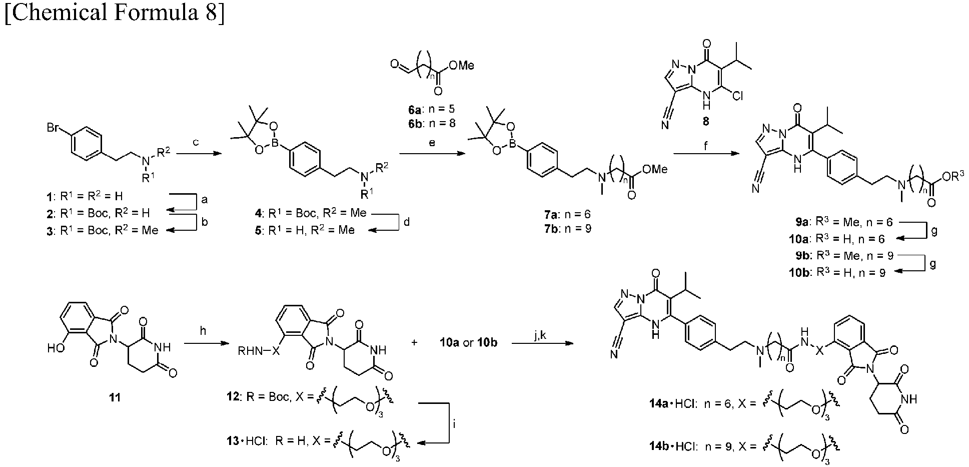

### <7-{[4-(3-Cyano-6-isopropyl-7-oxo-4,7-dihydropyrazolo[1,5-a]pyrimidin-5-yl)phenethyl](methyl)-amino}heptanoic Acid (10a) >

### Step 1: Synthesis of tert-butyl (4-bromophenethyl)carbamate (2)

Boc₂O (5.72 g, 26.2 mmol) was added to a solution of 2-(4-bromophenyl) ethylamine (Compound **1,** 5.00 g, 25.0 mmol) and triethylamine (4.20 mL, 30.1 mmol) in CH₂Cl₂ (50 mL) with cooling in an ice bath. The resulting mixture was stirred at room temperature for 5 h. Thereafter, the reaction was quenched by 10% citric acid and extracted with AcOEt. The organic layer was separated, washed with brine, and dried over MgSO₄. After filtration, concentration under reduced pressure, and purification *via* flash column chromatography (silica gel, *n*-hexane/AcOEt = 9/1 to 4/1), Compound **2** was obtained as a colorless solid (5.83 g, 78%).
¹H-NMR (DMSO-*d*₆, 400 MHz, *δ* ppm) 7.46 (2H, d, *J* = 8.3 Hz), 7.15 (2H, d, *J* = 8.3 Hz), 6.86 (1H, t, *J* = 5.2 Hz), 3.12 (2H, td, *J* = 6.8, 6.8 Hz), 2.66 (2H, t, *J* = 7.0 Hz), 1.35 (9H, s).

### Step 2: Synthesis of tert-Butyl (4-bromophenethyl)(methyl)carbamate (3)

A solution of Compound **2** (5.83 g, 19.4 mmol) in DMF (50 mL) was added to a suspension of 60% NaH in oil (0.820 g, 20.5 mmol) at 0 °C. After 15 min, MeI (1.40 mL, 22.5 mmol) was added to the mixture in a dropwise fashion, and the resulting mixture was stirred at 0 °C for 5 h. The reaction mixture was then poured into water and extracted with AcOEt. The organic layer was washed with brine and dried over MgSO₄.

After filtration, concentration under reduced pressure, and purification *via* flash column chromatography (silica gel, *n*hexane/AcOEt = 9/1), Compound **3** was obtained as a colorless oil (6.08 g, 99%).
¹H-NMR (DMSO-*d*₆, 400 MHz, *δ* ppm) 7.47 (2H, d, *J* = 8.1 Hz), 7.15 (2H, d, *J* = 8.1 Hz), 3.36 (2H, t, *J* = 6.5 Hz), 3.32 (3H, s), 2.72 (2H, t, *J* = 6.8 Hz), 1.25 (9H, s).

### Step 3: Synthesis of tert-butyl methyl[4-(4,4,5,5-tetramethyl-1,3,2-dioxaborolan-2-yl)phenethyl]carbamate (4)

A solution of Compound **3** (6.08 g, 19.3 mmol), bis(pinacolato)-diboron (5.70 g, 22.4 mmol), KOAc (7.30 g, 74.4 mmol), and Pd(dppf) Cl₂ (670 mg, 0.916 mmol) in DMSO (60 mL) was heated at 80 °C for 7 h. After the reaction mixture was filtered, the filtrate was extracted with AcOEt and washed with brine.

The organic layer was separated and dried over MgSO₄. After filtration, concentration under reduced pressure, and purification *via* flash column chromatography (silica gel, *n*-hexane/AcOEt = 4/1), Compound **4** was obtained as a colorless solid (5.76 g, 82%).
¹H-NMR (DMSO-*d*₆, 400 MHz, *δ* ppm), 7.59 (2H, d, *J* = 7.9 Hz), 7.21 (2H, d, *J* = 7.4 Hz), 3.36 (2H, t, *J* = 7.2 Hz), 2.76 (2H, t, *J* = 7.2 Hz), 2.73 (3H, s), 1.36 (9H, s), 1.28 (12H, s).

### Step 4: Synthesis of N-methyl-2-[4-(4,4,5,5-tetramethyl-1, 3, 2-dioxaborolan-2-yl)phenyl]ethan-1-amine (5)

Trifluoroacetic acid (TFA) (16 mL) was added to a solution of Compound **4** (5.76 g, 15.9 mmol) in CH2Cl2 (35 mL), which was cooled in an ice-bath. The resulting mixture was stirred at 0 °C for 2 h. The solvent was removed under reduced pressure, and saturated NaHCO₃ and AcOEt were added to the resulting residue and separated.

The organic layer was washed with brine, dried over MgSO₄, filtered, and the solvent removed under reduced pressure. The residue was purified *via* flash chromatography (silica gel, *n-*hexane/AcOEt = 4/1) to give Compound **5** as a colorless solid (3.50 g, 84%).
¹H-NMR (DMSO-*d*₆, 400 MHz, *δ* ppm), 8.45 (1H, br s), 7.65 (2H, d, *J* = 8.1 Hz), 7.28 (2H, d, *J* = 8.3 Hz), 3.15 (2H, t, *J* = 9.2 Hz), 2.94-2.89 (2H, m), 2.59 (3H, s), 1.28 (12H, s).

### Step 5: Synthesis of methyl 7-{methyl[4-(4,4,5,5-tetramethyl-1,3,2-dioxaborolan-2-l)phenethyl]amino}heptanoate (7a)

A solution of methyl 7-oxoheptanoate (Compound **6a**, 401 mg, 2.53 mmol), Compound **5** (632 mg, 2.42 mmol), and NaBH(OAc)₃ (820 mg, 3.87 mmol) in ClCH₂CH₂Cl (10 mL) was stirred at room temperature overnight. The reaction was quenched with water and extracted with AcOEt. The organic layer was separated, washed with brine, and dried over MgSO₄.

After filtration, concentration under reduced pressure, and purification *via* flash column chromatography (silica gel, CHCl₃/AcOEt = 4/1 to CHCl₃/MeOH = 9/1), Compound **7a** was obtained as a colorless oil (253 mg, 26%).
¹H-NMR (DMSO*d*₆, 400 MHz, *δ* ppm), 7.57 (2H, d, *J* = 8.8 Hz), 7.22 (2H, d, *J* = 8.8 Hz), 3.57 (3H, s), 2.74-2.64 (2H, m), 2.35-2.23 (4H, m), 2.17 (3H, br s), 1.55-1.43 (2H, m), 1.41-1.32 (2H, m), 1.28 (12H, s), 1.25-1.14 (6H, m).

### Step 6: Synthesis of methyl 7-{[4-(3-cyano-6-isopropyl-7-oxo-4,7-dihydropyrazolo[1,5-a]pyrimidin-5-yl)phenethyl](methyl)amino}heptanoate (9a)

A solution of Compound **7a** (147 mg, 0.364 mmol), Compound **8** (78.3 mg, 0.331 mmol), Na₂CO₃ (70.2 mg, 0.662 mmol), and Pd(dppf) Cl₂ (24.2 mg, 0.0331 mmol) in DME/H₂O (1.5 mL/0.5 mL) was heated at 110 °C for 3 h. After the reaction mixture was filtered, the filtrate was extracted with AcOEt and washed with brine.

The organic layer was separated and dried over MgSO₄. After filtration, concentration under reduced pressure, and purification *via* flash column chromatography (silica gel, CHCl₃/AcOEt = 4/1 to CHCl₃/MeOH = 9/1), Compound **9a** was obtained as a colorless solid (23.5 mg, 15%). ¹H-NMR (DMSO-*d*₆, 400 MHz, *δ* ppm), 8.03 (1H, br s), 7.39 (2H, d, *J* = 7.9 Hz), 7.32 (2H, d, *J* = 7.9 Hz), 3.57 (3H, s), 2.88-2.76 (4H, m), 2.60-2.53 (3H, m), 2.38 (3H, s), 2.28 (2H, t, *J* = 7.2 Hz), 1.54-1.42 (4H, m), 1.30-1.23 (4H, m), 1.18 (6H, d, *J* = 8.2 Hz); MS (ESI) *m*/*z* 478 (MH+).

### Step 7: Synthesis of 7-{[4-(3-cyano-6-isopropyl-7-oxo-4,7-dihydropyrazolo[1,5-a]pyrimidin-5-yl)phenethyl](methyl)amino}heptanoic acid (10a)

An aqueous solution of NaOH (4N, 0.200 mL, 0.791 mmol) was added to a solution of Compound **9a** (126 mg, 0.264 mmol) in MeOH (1.2 mL), and the mixture was stirred at room temperature overnight. 10% aqueous citric acid was added portion-wise, and the resulting mixture was stirred at room temperature. The insoluble material was collected *via* filtration and washed with water to yield Compound **10a** as a colorless solid (66.0 mg, 54%).
¹H-NMR (DMSO-*d*₆, 400 MHz, *δ* ppm), 11.9 (1H, br s), 9.24 (1H, br s), 8.02 (1H, s), 7.33 (4H, d, *J* = 9.6 Hz), 3.11-2.92 (2H, m), 2.90-2.78 (2H, m), 2.75-2.68 (1H, m), 2.52-2.50 (2H, m), 2.51 (3H, s), 2.21 (2H, *t*, *J* = 7.3 Hz), 1.68-1.60 (2H, m), 1.54-1.48 (2H, m), 1.36-1.27 (4H, m), 1.23 (6H, d, *J* = 6.9 Hz); MS (ESI) *m*/*z* 464 (MH+).

### < Synthesis of 7-{[4-(3-Cyano-6-isopropyl-7-oxo-4,7-dihydropyrazolo[1,5-a]pyrimidin-5-yl)phenethyl](methyl)-amino}-N-{2-[2-(2,6 -dioxopiperidin-3-yl)-1, 3-dioxoindolin-4-yl]oxy}ethoxy)ethyl}heptanamide Hydrochloride (14a·HCl)>

### Step 1: Synthesis of {2-[2-(2-{[2-(2,6-dioxopiperidin-3-yl)-1,3-dioxoisoindolin-4-yl]oxy}ethoxy)ethoxy]ethyl}carbamate (12)

2-[2-(2-*tert*-Butoxyaminoethoxy) ethoxy]- ethanol (300 mg, 1.20 mmol) and DMEAD (383 mg, 1.64 mmol) were added to a solution of 2-(2,6-dioxopiperidin-3-yl)-4- hydroxyisoindoline-1,3-dione (Compound **11**, 300 mg, 1.09 mmol), and PPh₃ (430 mg, 1.64 mmol) in THF (3 mL) at room temperature. The reaction mixture was stirred at room temperature overnight. The reaction was quenched by water and extracted with AcOEt.

The organic layer was separated and dried over MgSO₄. After filtration, concentration under reduced pressure, and purification *via* flash column chromatography (silica gel, CHCl₃/AcOEt = 4/1 to 1/1), Compound **12** was obtained as a colorless oil (226 mg, 41%).
¹H-NMR (DMSO-*d*₆, 400 MHz, *δ* ppm), 11.10 (1H, brs), 7.85-7.78 (1H, m), 7.54 (1H, d, *J* = 7.8 Hz), 7.46 (1H, d, *J* = 7.4 Hz), 6.74 (1H, brs), 5.08 (1H, dd, *J* = 12.8, 5.4 Hz), 4.35 (2H, t, *J* = 4.5 Hz), 3.80 (2H, t, *J* = 4.5 Hz), 3.64 (2H, t, *J* = 4.7 Hz), 3.53-3.47 (2H, m), 3.40-3.36 (2H, m), 3.08-3.01 (2H, m), 2.94-2.84 (1H, m), 2.62-2.51 (2H, m), 2.05-2.01 (1H, m), 1.36 (9H, s).

### Step 2: Synthesis of 4-(2-(2-aminoethoxy)ethoxy)-2-(2,6-dioxopiperidin-3-yl)isoindoline-1,3-dione Hydrochloride (13·HCl)

Trifluoroacetic acid (TFA) (0.34 mL) was added to a solution of Compound **12** (226 mg, 0.448 mmol) in CH₂Cl₂ (2 mL), with cooling in an ice bath. After being stirred at 0 °C for 3 h, the reaction mixture was concentrated under reduced pressure. 4N HCl in AcOEt was added to the residue, and the solvent was removed under reduced pressure to yield Compound **13·HCl** (239 mg) as a crude product, which was used in the next reaction without further purification.

### Step 3: Synthesis of 7-{[4-(3-cyano-6-isopropyl-7-oxo-4,7-dihydropyrazolo[1,5-a]pyrimidin-5-yl)phenethyl](methyl)amino}-N-{2-[2- (2,6-dioxopiperidin-3-yl)-1,3-dioxoindolin-4-yl]oxy}ethoxy}ethyl}heptanamide (14a)

HATU (20.0 mg, 0.0526 mmol) was added to a solution of Compound **10a** (19.9 mg, 0.0429 mmol), *i*-Pr₂NEt (0.0180 mL, 0.104 mmol), and crude Compound **13·HCl** (19.0 mg) in DMF (0.2 mL) at room temperature. The reaction mixture was stirred at room temperature overnight. Water was added to this reaction, followed by extraction with AcOEt twice. The combined organic layer was washed with brine, dried over MgSO₄, filtered, and concentrated. The filtrate was concentrated and purified *via* reverse phase flash chromatography (MeCN/0.1%TFA) to obtain Compound **14a**, which was converted to a hydrochloride salt in the next step.

### Step 4: Synthesis of 7-{[4-(3-cyano-6-isopropyl-7-oxo-4,7-dihydropyrazolo[1,5-a]pyrimidin-5-yl)phenethyl](methyl)amino}-N-{2-[2-(2,6-dioxopiperidin-3-yl)-1,3-dioxoindolin-4-yl]oxy}ethoxy}ethyl}heptanamide hydrochloride (14a·HCl)

Compound **14a** was treated with 4N HCl in AcOEt, and the solvent was removed under reduced pressure to yield Compound **14a·HCl** (5.8 mg, 15% from Compound **10a**) as a colorless solid.
¹H-NMR (DMSO-*d*₆, 700 MHz, *δ* ppm), *δ* 13.44 (1H, s), 11.13 (1H, s), 8.41 (1H, s), 7.88 (1H, br s), 7.81 (1H, t, *J* = 8.4 Hz), 7.54 (1H, d, *J* = 8.6 Hz), 7.50-7.48 (4H, m), 7.47 (1H, d, *J* = 7.0 Hz), 5.09 (1H, dd, *J* = 12.9, 5.6 Hz), 4.34 (2H, t, *J* = 4.3 Hz), 3.81-3.80 (2H, m), 3.66-3.64 (2H, m), 3.53-3.52 (2H, m), 3.41-3.39 (2H, m), 3.32-3.25 (1H, m), 3.19-2.99 (6H, m), 2.91-2.80 (1H, m), 2.82 (3H, s), 2.60-2.58 (2H, m), 2.55-2.49 (2H, m), 2.08-2.06 (2H, m), 2.04-2.00 (1H, m), 1.71-1.63 (2H, m), 1.51-1.46 (2H, m), 1.31-1.25 (4H, m), 1.23 (6H, d, *J* = 7.0 Hz, 6H); ¹³C-NMR (DMSO-*d*₆, 175 MHz, *δ* ppm), 172.22, 171.48, 169.37, 166.20, 164.69, 155.18, 154.40, 147.27, 144.73, 142.85, 138.43, 136.42, 132.62, 130.91, 128.34, 128.25, 119.38, 115.65, 114.81, 113.38, 112.16, 73.37, 69.48, 68.99, 68.56, 68.23, 68.06, 54.83, 54.27, 48.11, 37.80, 34.47, 30.33, 28.60, 27.75, 27.53, 25.20, 24.35, 22.47, 21.37, 20.47, 19.34; HRMS Calcd for C₄₅H₅₅N₈O₉ + 851.4092, Found 851.4077; HPLCRₜ 12.27 min, 97.84% purity.

### <<Example 2: Synthesis of 10-{[4-(3-Cyano-6-isopropyl-7-oxo-4,7-dihydropyrazolo[1,5-a]pyrimidin-5-yl)phenethyl](methyl)-amino}-N-{2-[2-(2,6 -dioxopiperidin-3-yl)-1, 3-dioxoindolin-4-yl]oxy}ethoxy}ethyl}decanamide Hydrochloride (14b·HCl) >>

Compound **14b·HCl** was prepared using procedures similar to that described for the synthesis of Compound **10a** and Compound **14a·HCl** except that methyl 10-oxodecanoate (6b) was used instead of methyl 7-oxoheptanoate (6a); yellow solid (5.9 mg, 16% from Compound **10b**);
¹H-NMR (DMSO-*d*₆, 700 MHz, *δ* ppm), 13.43 (1H, s), 11.14 (1H, s), 8.42 (1H, s), 7.84-7.81 (2H, m), 7.54 (1H, d, *J* = 8.6 Hz), 7.50 (4H, s), 7.47 (1H, d, *J* = 7.3 Hz), 5.10 (1H, dd, *J* = 12.9, 5.2 Hz), 4.35 (2H, t, *J* = 4.3 Hz), 3.81 (2H, t, *J* = 4.5 Hz), 3.65 (2H, t, *J* = 4.7 Hz), 3.53 (2H, t, *J* = 4.7 Hz), 3.50-3.35 (2H, m), 3.32-3.29 (1H, m), 3.22-3.04 (6H, m), 2.94-2.86 (1H, m), 2.85 (3H, s), 2.61-2.57 (2H, m), 2.55-2.48 (2H, m), 2.06-2.02 (1H, m), 2.04 (2H, t, *J* = 7.0 Hz), 1.72-1.60 (2H, m), 1.48-1.44 (2H, m), 1.29-1.23 (10H, m), 1.23 (6H, d, *J* = 7.0 Hz); ¹³C-NMR (DMSO-*d*₆, 175 MHz, *δ* ppm), 172.21, 171.54, 169.35, 166.19, 164.67, 157.35, 157.16, 155.18, 154.39, 147.23, 144.72, 142.84, 138.27, 136.41, 132.62, 130.94, 128.34, 128.25, 119.36, 115.64, 114.80, 113.36, 112.16, 73.36, 69.47, 68.99, 68.56, 68.22, 68.06, 54.87, 54.42, 52.88, 48.10, 41.15, 37.79, 34.63, 30.32, 28.64, 28.11, 28.07, 28.02, 27.88, 27.75, 25.35, 24.60, 22.67, 21.36, 20.45, 19.34, 17.43, 16.09, 11.84; HRMS Calcd for C₄₈H₆₁N₈O₉⁺ 893.4562, Found 893.4545; HPLC Rₜ 13.26 min, 95.59% purity.

### <<Example 3: Synthesis of 4-(6-{[4-(3-Cyano-6-isopropyl-7-oxo-4,7-dihydropyrazolo[1,5-a]pyrimidin-5-yl)phenethyl](methyl)-amino}hexyl)-N-{2-[2- (2,6-dioxopiperidin-3-yl)-1,3-dioxoindolin-4-yl]oxy}ethoxy)ethoxy]ethyl}benzamide (20a) >>

Compound 20a was synthesized according to the following reaction scheme.

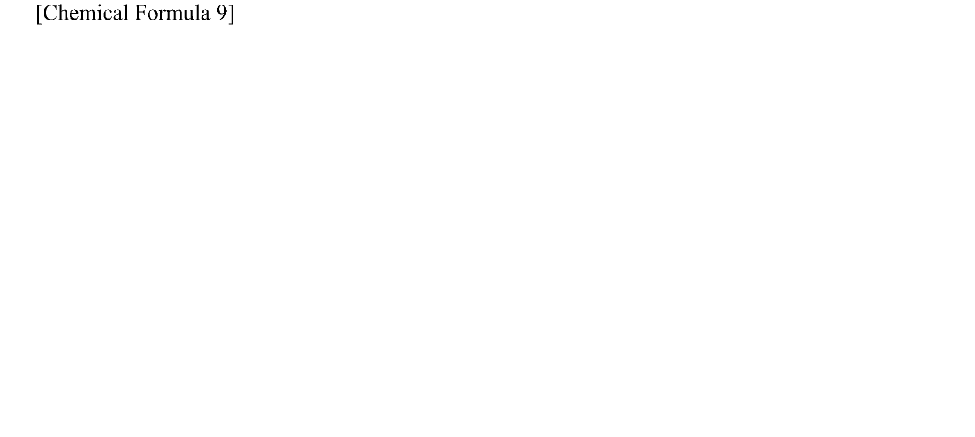

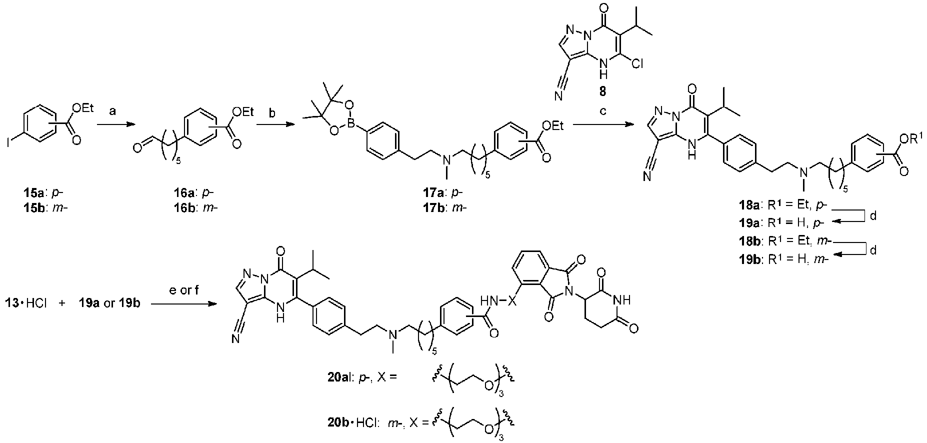

### Step 1: Synthesis of ethyl 4-(6-oxohexyl)benzoate (16a)

A solution of Compound **15a** (2.42 g, 8.77 mmol), 5-hexen-1-ol (1.05 g, 10.5 mmol), LiOAc (1.44 g, 21.8 mmol), LiCl (371 mg, 8.75 mmol), tetrabutylammonium chloride (1.22 g, 4.39 mmol), and Pd(OAc)₂ (195 mg, 0.869 mmol) in DMF (10 mL) was heated at 70 °C for 6.5 h. After the reaction mixture was filtered, the filtrate was extracted with AcOEt and washed with brine.

The organic layer was separated and dried over MgSO₄. After filtration, concentration under reduced pressure, and purification *via* flash column chromatography (silica gel, n-hexane/AcOEt = 4/1), Compound **16a** was obtained as a colorless oil (1.96 g, 90%).
¹H-NMR (DMSO-*d*₆, 400 MHz, *δ* ppm), 9.65 (1H, t, *J =* 1.5 Hz), 7.87 (2H, d, *J =* 8.4 Hz), 7.34 (2H, d, *J* = 8.4 Hz), 4.29 (2H, q, *J* = 7.1 Hz), 2.64 (2H, t, *J* = 7.6 Hz), 2.41 (2H, td, *J* = 7.0, 2.0 Hz), 1.63-1.51 (4H, m), 1.33-1.26 (2H, m), 1.31 (3H, t, *J* = 7.2 Hz).

### Step 2-4: Synthesis of 4-(6-{[4-(3-Cyano-6-isopropyl-7-oxo-4,7-dihydropyrazolo[1,5-a]pyrimidin-5-yl)phenethyl] (methyl)amino}hexyl)benzoic Acid (19a)

Compound **19a** was prepared from Compound **8** and Compound **16a** using procedures similar to that described for the synthesis of Compound **10a** (steps 5-7); yellow solid (25.0 mg, 7% from Compound **16a).**
¹H-NMR (DMSO-*d*₆, 400 MHz, *δ* ppm), 12.8 (1H, brs), 8.01 (1H, brs), 7.86 (2H, d, *J* = 7.8 Hz), 7.33-7.31 (6H, m), 3.33-3.24 (1H, m), 3.06-2.93 (4H, m), 2.80 (3H, br s), 2.68-2.64 (4H, m), 1.67-1.56 (4H, m), 1.37-1.29 (4H, m), 1.24 (6H, d, *J=* 9.8 Hz); MS (ESI) *m*/*z* 540 (MH⁺).

### Step 5: Synthesis of 4-(6-{[4-(3-cyano-6-isopropyl-7-oxo-4,7-dihydropyrazolo[1, 5-a]pyrimidin-5-yl) phenethyl](methyl)amino}hexyl)-N-{2-[2- (2,6 -dioxopiperidin-3-yl)-1,3-dioxoindolin-4-yl]oxy}ethoxy) ethoxy]ethyl}benzamide (20a)

Compound **20a** was prepared from Compound **13·HCl** and Compound **19a** using a procedure similar to that described for the synthesis of **14a·HCl** (step 3); yellow solid (9.1 mg, 21%). ¹H-NMR (DMSO-*d*₆, 700 MHz, *δ* ppm), 11.14 (1H, s), 8.44 (1H, t, *J =* 5.6 Hz), 8.04 (1H, s), 7.82-7.76 (3H, m), 7.52 (1H, d, *J =* 7.0 Hz), 7.47 (1H, d, *J =* 7.0 Hz), 7.32-7.26 (6H, m), 5.10 (1H, dd,*J* = 12.9, 5.2 Hz), 4.33-4.23 (2H, m), 3.84-3.80 (2H, m), 3.66 (2H, t, *J* = 4.7 Hz), 3.57-3.53 (4H, m), 3.43-3.30 (6H, m), 2.92-2.87 (2H, m), 2.74 (1H t, *J* = 6.5 Hz), 2.65-2.55 (5H, m), 2.54-2.48 (2H, m), 2.04-2.00 (1H, m), 1.63-1.46 (4H, m), 1.35-1.28 (4H, m), 1.30 (6H, d, *J* = 18.1 Hz); ¹³C-NMR (DMSO*d*₆, 175 MHz,*δ* ppm), 172.70, 169.85, 166.69, 166.00, 165.16, 156.73, 155.67, 145.54, 143.81, 136.87, 133.10, 131.75, 128.00, 127.94, 127.16, 127.07, 126.61, 124.80, 119.83, 116.14, 115.26, 110.56, 73.97, 69.95, 69.55, 68.81, 68.68, 68.54, 59.65, 48.59, 34.72, 34.27, 30.85, 30.82, 30.47, 30.28, 29.11, 28.72, 28.22, 21.96, 21.86, 20.66, 20.44, 13.87; HRMS Calcd for C₅₁H₅₉N₈O₉⁺ 927.4405, Found 927.4390; HPLCRₜ 13.73 min, 95.89% purity.

### <<Example 4: Synthesis of 4-(6-{[4-(3-Cyano-6-isopropyl-7-oxo-4,7-dihydropyrazolo[1,5-a]pyrimidin-5-yl)phenethyl](methyl)-amino}hexyl)-N-{2-[2- (2,6-dioxopiperidin-3-yl)-1,3-dioxoindolin-4-yl]oxy}ethoxy)ethoxy]ethyl}benzamide Hydrochloride (20b·HCl)>>

Compound **20b·HCl** was prepared using procedures similar to that described for the synthesis of Compound **20a** (steps 1-5) and Compound **14a·HCl** (step 4) except that ethyl 3-(6-oxohexyl) benzoate (**16b**) was used instead of ethyl 4-(6-oxohexyl) benzoate (**16a**); yellow solid (16.6 mg, 17% from Compound **8**). Compound **16b** was synthesized using the same procedure as described for the synthesis of Compound **16a** (Step 1), except that Compound **15b** was used instead of Compound **15a**.
¹H-NMR (DMSO-*d*₆, 700 MHz, *δ* ppm), 13.44 (1H, s), 11.15 (1H, d, *J* = 5.2 Hz, 1H), 8.51 (1H, t, *J* = 5.6 Hz), 8.43 (1H, s), 7.82 (2H, t, *J* = 8.0 Hz), 7.74-7.67 (2H, m), 7.53-7.47 (6H, m), 7.39-7.36 (2H, m), 5.10 (1H, dd, *J* = 12.9, 5.2 Hz), 4.33 (2H, t, *J* = 4.5 Hz), 3.81 (2H, t, *J* = 4.5 Hz), 3.67 (2H, t, *J* = 4.7 Hz), 3.58 (2H, t, *J* = 4.7 Hz), 3.53 (2H, t, *J* = 4.7 Hz), 3.52-3.37 (6H, m), 3.35-3.27 (1H, m), 3.22-3.00 (4H, m), 2.92-2.87 (1H, m), 2.86 (3H, s), 2.65-2.59 (4H, m), 2.55-2.48 (2H, m), 2.04-2.01 (1H, m), 1.70-1.66 (2H, m), 1.66-1.63 (2H, m), 1.40-1.32 (4H, m), 1.25 (6H, d, *J* = 7.0 Hz); ¹³C-NMR (DMSO-*d*₆, 175 MHz, *δ* ppm), 172.71, 171.93, 169.86, 166.69, 166.20, 165.17, 155.66, 154.89, 147.74, 145.22, 143.34, 142.15, 138.82, 136.88, 134.24, 133.11, 131.42, 130.98, 128.83, 128.74, 128.04, 127.05, 124.40, 119.84, 116.14, 115.28, 113.86, 112.66, 73.86, 69.96, 69.53, 68.78, 68.69, 68.54, 55.33, 54.83, 48.59, 34.77, 30.82, 30.49, 29.12, 28.25, 28.00, 25.71, 23.07, 22.02, 21.85, 20.96, 19.84; HRMS Calcd for C₅₁H₅₉N₈O₉⁺ 927.4405, Found 927.4388; HPLC Rₜ 13.80 min, 97.80% purity.

### <<Example 5: Synthesis of 4-(6-{[4-(3-Cyano-6-isopropyl-7-oxo-4,7-dihydropyrazolo[1,5-a]pyrimidin-5-yl)phenethyl](methyl)-amino}hexyl)-N-(9-{[2-(2,6-dioxopiperidin-3-yl)-1,3-dioxoindolin-4-yl]oxy}nonyl)benzamide Hydrochloride (23a·HCl) >>

Compound **23a**-hydrochloride was synthesized according to the following reaction scheme.

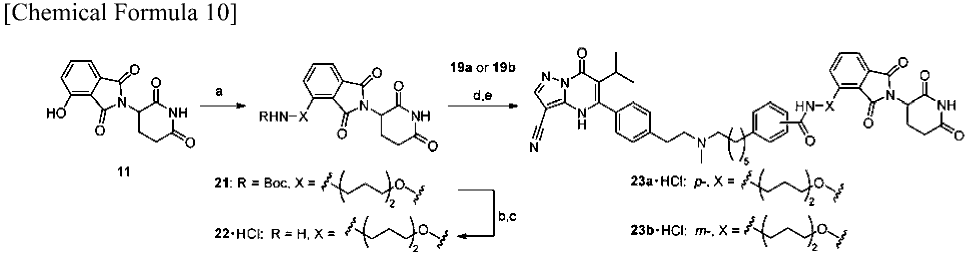

### Step 1: Synthesis of tert-butyl (6-{[2-(2,6-dioxopiperidin-3-yl)-1,3-dioxoisoindolin-4-yl]oxy}hexyl)carbamate (21)

A solution of Compound **11** (274 mg, 1.00 mmol), *tert*-butyl (6-bromohexyl) carbamate (280 mg, 1.00 mmol), KHCO₃ (150 mg, 1.50 mmol), and KI (17.0 mg, 0.102 mmol) in DMF (3 mL) was heated at 80 °C for 14 h.

After cooling to room temperature, water and AcOEt were added and separated. The organic layer was washed with brine, dried over MgSO₄. After filtration, concentration under reduced pressure, and purification *via* flash column chromatography (CHCl₃/ AcOEt = 10/1 to 1/1), Compound **21** was obtained as a colorless oil (373 mg, 79%).
¹H-NMR (DMSO-*d*₆, 400 MHz, *δ* ppm), 11.08 (1H, brs), 7.82-7.78 (1H, m), 7.51 (1H, d, *J* = 8.2 Hz), 7.44 (1H, d, *J* = 7.6 Hz), 6.75 (1H, br s), 5.07 (1H, dd, *J* = 12.8, 5.4 Hz), 4.20 (2H, t, *J* = 6.5 Hz), 2.93-2.90 (1H, m), 2.93-2.83 (2H, m), 2.61-2.53 (2H, m), 2.04-2.00 (1H, m), 1.78-1.71 (2H, m), 1.49-1.26 (6H, m), 1.36 (9H, s).

### Step 2: Synthesis of 4-[(6-aminohexyl)oxy)-2-(2,6-dioxopiperidin-3-yl)isoindoline-1,3-dione hydrochloride (22·HCl)

Compound **22·HCl** was prepared as a crude product (404 mg) using a procedure similar to that described for the preparation of Compound **14a·HCl** (step 2). This was used directly in the next reaction without further purification.

### Step 3: Synthesis of 4-(6-{[4-(3-cyano-6-isopropyl-7-oxo-4,7-dihydropyrazolo[1,5-a]pyrimidin-5-yl)phenethyl](methyl)amino}hexyl)-N-(6-{[2-(2,6-dioxopiperidin-3-yl) -1, 3 - dioxoindolin-4-yl]oxy}hexyl) benzamide hydrochloride (23a·HCl)

Compound **23a·HCl** was prepared from Compound **19a** and Compound **22·HCl** using procedures similar to that described for the synthesis of Compound **14a·HCl** (steps 3 and 4); yellow solid (26.0 mg, 52%).
¹H-NMR (DMSO-*d*₆, 700 MHz, *δ* ppm), 13.43 (1H, s), 11.12 (1H, s), 8.42 (1H, s), 8.41-8.38 (1H, m), 7.81 (1H, dd, *J* = 7.0, 7.0 Hz), 7.76 (2H, d, *J* = 8.2 Hz), 7.52 (1H, d, *J* = 8.6 Hz), 7.50-7.48 (4H, m), 7.44 (1H, d, *J* = 6.9 Hz), 7.27 (2H, d, *J* = 8.2 Hz), 5.08 (1H, dd, *J* = 12.9, 5.6 Hz), 4.21(2H, t, *J* = 6.5 Hz), 3.32-3.28 (1H, m), 3.27-3.24 (2H, m), 3.20-2.99 (4H, m), 2.91-2.86 (1H, m), 2.83 (3H, s), 2.65-2.61 (2H, m), 2.60-2.56 (2H, m), 2.53-2.47 (2H, m), 2.03-2.00 (1H, m), 1.79-1.75 (2H, m), 1.73-1.65 (2H, m), 1.65-1.57 (2H, m), 1.57-1.52 (2H, m), 1.52-1.46 (2H, m), 1.41-1.36 (2H, m), 1.36-1.31 (4H, m), 1.24 (6H, d, *J* = 7.0 Hz); ¹³C-NMR (DMSO*d*₆, 175 MHz, *δ* ppm), 172.20, 169.37, 166.23, 165.28, 164.70, 155.35, 154.38, 147.23, 144.76, 144.71, 142.83, 136.42, 132.60, 131.60, 130.92, 128.32, 128.23, 127.47, 126.55, 126.08, 119.12, 115.54, 114.51, 113.35, 112.14, 73.35, 68.08, 54.82, 54.35, 48.06, 34.14, 30.31, 29.83, 28.62, 28.50, 27.74, 27.48, 25.53, 25.19, 24.45, 22.58, 21.35, 20.45, 19.33; HRMS Calcd for C₅₁H₅₉N₈O₇⁺ 895.4507, Found 895.4490; HPLC Rₜ 15.16 min, 95.44% purity.

### <<Example 6: Synthesis of 3-(6-{[4-(3-Cyano-6-isopropyl-7-oxo-4,7-dihydropyrazolo[1,5-a]pyrimidin-5-yl)phenethyl](methyl)-amino}hexyl)-N-(6-{[2-(2,6-dioxopiperidin-3-yl)-1,3-dioxoindolin-4-yl]oxy}hexyl)benzamide Hydrochloride(23b·HCl) >>

Compound **23b·HCl** was prepared using procedures similar to that described for the synthesis of Compound **23a·HCl** (steps 1-3); yellow solid (10.4 mg, 11%);
¹H-NMR (DMSO-*d*₆, 700 MHz, *δ* ppm), 13.43 (1H, s), 11.12 (1H, s), 8.47-8.41 (2H, m), 7.80 (1H, dd, *J* = 8.0, 8.0 Hz), 7.71-7.64 (2H, m), 7.52-7.43 (6H, m), 7.36-7.34 (2H, m), 5.08 (1H, dd, *J* = 12.9, 5.6 Hz), 4.20 (2H, t, *J* = 6.5 Hz), 3.33-3.30 (1H, m), 3.28-3.24 (2H, m), 3.17-3.00 (4H, m), 2.90-2.85 (1H, m), 2.82 (3H, s), 2.65-2.61 (2H, m), 2.61-2.56 (2H, m), 2.54-2.47 (2H, m), 2.04-2.00 (1H, m), 1.79-1.75 (2H, m), 1.73-1.66 (2H, m), 1.65-1.59 (2H, m), 1.57-1.52 (2H, m), 1.52-1.47 (2H, m), 1.41-1.37 (2H, m), 1.37-1.31 (4H, m), 1.23 (6H, d, *J* = 7.0 Hz); ¹³C-NMR (DMSO*d*₆, 175 MHz, *δ* ppm), 172.20, 169.37, 166.22, 165.51, 164.71, 155.35, 141.62, 136.42, 134.06, 132.60, 130.31, 128.30, 128.20, 127.49, 126.50, 123.87, 119.12, 115.54, 114.52, 73.35, 68.08, 54.80, 54.28, 48.06, 34.28, 30.31, 30.00, 28.59, 28.47, 27.74, 27.50, 25.55, 25.21, 24.45, 23.62, 22.52, 21.52, 21.45, 21.35, 20.45, 19.34, 13.36; HRMS Calcd for C₅₁H₅₉N₈O₇⁺ 895.4507, Found 895.4494; HPLC Rₜ 15.26 min, 95.66% purity.

### (2) Neurite outgrowth assay

The Mouse Neuro-2a (N2a) cell line was obtained from the Japanese Collection of Research Bioresources (JCRB) Cell Bank. The N2a cells were plated at a concentration of 1 × 10⁴ cells/mL in Dulbecco's modified Eagle's medium (hereinafter also referred to as DMEM) containing high glucose, 10% FBS, 100 U/mL penicillin, and 100 *µ*g/mL streptomycin at 37 °C in a humidified atmosphere of 5% CO₂.

For the neurite outgrowth assay, the medium was changed to DMEM supplemented with 2% FBS. After incubation with the test compounds, cell morphology was examined using a microscope (Olympus CKX41).

The differentiated cells were defined as those with at least one neurite that was longer than twice the diameter of the cell body. The results were expressed as the percentage of differentiated cells among the counted cells. These experiments were carried out in triplicate.

### (3) Western blotting

The N2a cells (5 × 10⁵ cells/2 mL/dish) were treated for 24 h with the test compounds at the indicated concentrations in a 10% FBS-supplemented cell culture medium, and cells were collected and extracted with SDS sampling buffer. Protein concentrations of the lysates were determined using a BCA protein assay. Equivalent amounts of protein from each lysate were resolved in 5% to 20% SDS-polyacrylamide gels, and bands were transferred onto PVDF membranes (Millipore, #IPVH00010 for *β*-actin detection, #ISEQ10100 for H3, H3K4me3, and H3K27Ac detection). After blocking with TBS-T containing 5% skimmed milk, the transblotted membranes were probed with primary antibodies. The following antibodies were used as the primary antibodies: rabbit monoclonal KDM5A antibody (CST #3876, 1 : 1000 dilution); mouse monoclonal *β*-actin antibody (Santa Cruz, #sc-47778, 1 : 2000 dilution); rabbit polyclonal H3K4me3 antibody (Abcam, #ab8580, 1 : 5000 dilution); rabbit polyclonal H3K27Ac antibody (Abcam, #ab4729, 1 : 1000 dilution); or rabbit polyclonal histone H3 antibody (Abcam, #ab1791, 1 : 200000 dilution). The probed membranes were washed thrice with TBS-T; incubated with ECL rabbit IgG HRP-linked whole antibody (GE Healthcare Life Science, #NA934, 1 : 2500 dilution), or ECL mouse IgG HRP-linked whole antibody (GE Healthcare Life Science, #NA931, 1 : 2500 dilution); and again, washed thrice with TBS-T. The immunoblots were visualized using enhanced chemiluminescence with chemiluminescent HRP substrate (Millipore, #P90718).

### (4) Results and Discussion

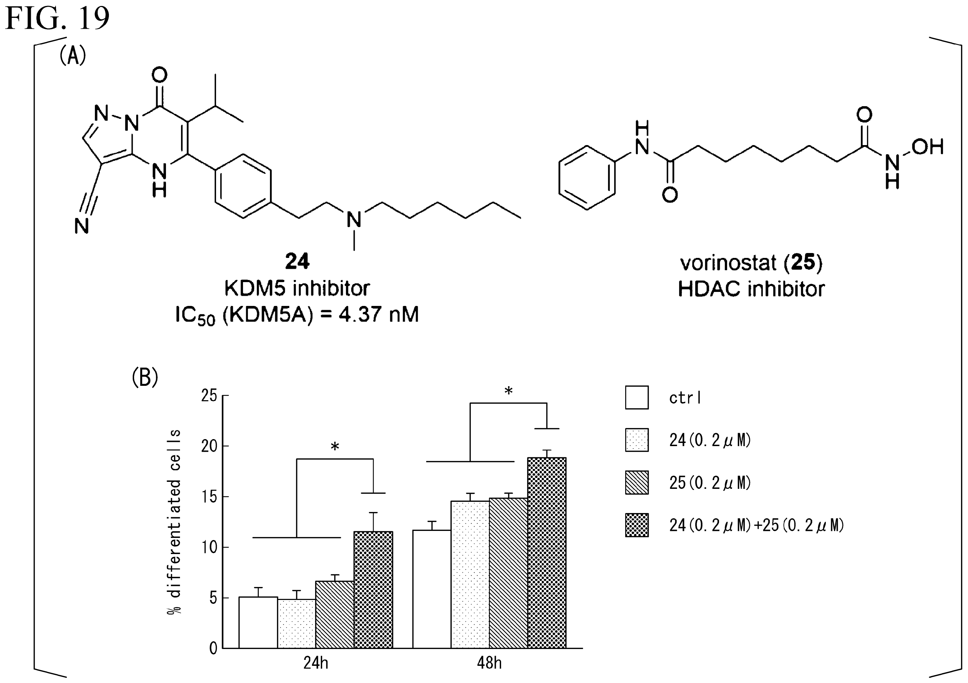

Before investigating KDM5 PROTACs, we tested Compound **24** (Fig. 19(A)) in a neurite outgrowth assay, which is often used for early screening in the drug discovery of neurodegenerative disorders, because compounds that inhibit KDM5A are suggested to be therapeutic candidates for neurodegenerative disorders, including Alzheimer's disease. As shown in Fig. 19(B), treatment of mouse neuroblastoma N2a cells with 0.2 *µ*M of Compound **24** for 24 or 48 h incubation did not strongly induce neurite outgrowth. This indicates that conventional KDM5 inhibitors, which inhibit only the catalytic function of KDM5A, do not exhibit strong neurite outgrowth activity. Based on the assumption that the entire functionality of KDM5A, including the scaffold that interacts with HDAC1 and 2 to control histone deacetylation, is important for neurite outgrowth, we examined the neurite outgrowth-promoting activity of the KDM5 inhibitor with the HDAC inhibitor vorinostat43) (**25**) (Fig. 19(A)). Notably, this combination significantly induced neurite outgrowth compared to Compound **24** or Compound **25** alone (Fig. 19(B)). This indicates that two approaches, inducing both histone methylation and acetylation through the inhibition of KDM5A and HDAC, respectively, is an effective means of promoting neurite outgrowth. Based on these results, we envisioned that KDM5A degradation by TPD (target protein degradation) would also strongly induce neurite outgrowth of N2a cells, as protein degradation would disrupt both its catalytic and scaffolding functions. This idea prompted us to prepare KDM5 PROTAC candidates that could show potent neurite outgrowth activity.

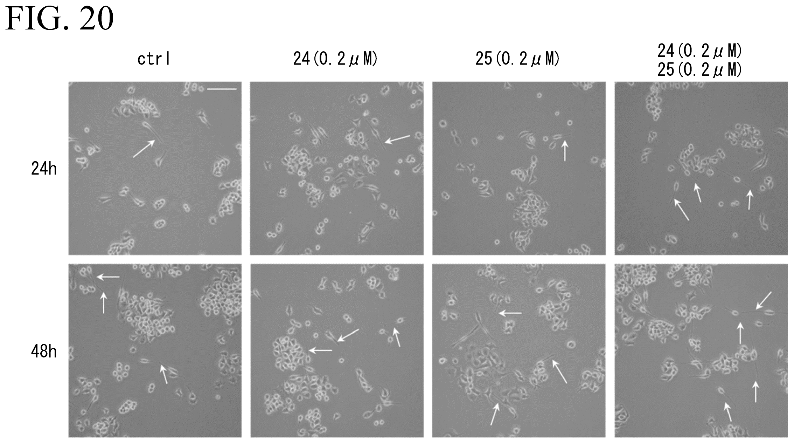

Figure 19(A) shows the structure of KDM5 inhibitor **24** and vorinostat (**25**), and Figure 19(B) shows the effect of single treatment with Compounds **24** and **25** and the effect of co-treatment with Compounds **24** and **25**, on N2a differentiation after 24 and 48 h treatment. Bars represent the mean values ± SD from three independent experiments. The p-values were determined using Tukey's multiple comparison test; *p < 0.05. Representative images showing N2a cells treated with Compounds **24** and **25** alone, as well as N2a cells co-treated with compounds **24** and **25**, are shown in Figure 20.

To design KDM5 PROTAC, we chose Compound **24** as a KDM5 ligand. Simulations of Compound **24** bound to KDM5A suggested that the hexyl chain is directed towards the protein surface. Based on this simulation, we introduced a linker at the terminal of the hexyl group.

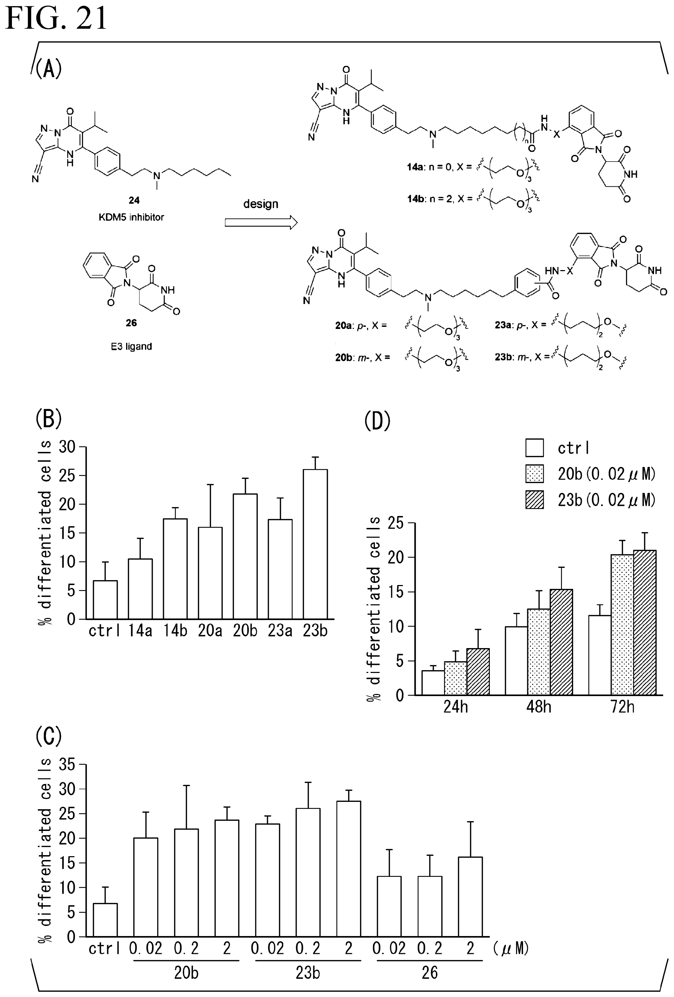

As the linker structure often affects degradation activities, we tried a number of different linkers, such as amide-conjugated polyethylene glycol linkers (**14**) and benzene ring-containing linkers (**20** and **23**) (Fig. 21(A)). Additionally, we selected thalidomide (**26**) as an E3 ligand, which is the most widely utilized for PROTAC studies.

Next, we prepared Compounds **14a, 14b**, **20a, 20b**, **23a** and **23b** as novel KDM5 PROTAC candidates (Fig. 21(A)) (see chemical formula above) and then screened the synthetic compounds in the neurite outgrowth activity assay. As shown in Fig. 21 (B), treatment of N2a cells with compounds **14a, 14b**, **20a, 20b**, **23a** and **23b** promoted neurite outgrowth. Compound **14b** showed a stronger neurite outgrowth-promoting activity than compound **14a**. This indicates that the introduction of a spacer between the hexyl group of the KDM5 inhibitor and the amide group of the linker is important for neurite outgrowth-promoting activity. Moreover, the replacement of the spacer with a benzene ring, which was expected to contribute to the rigidity of linker orientation, maintained or increased its effectiveness. Importantly, the activities of the *meta*-substituent compounds **20b** and **23b** were superior to those of the *para*-substituent compounds **20a** and **23a**. Furthermore, the activity of the carbon linker compound **23b** was slightly better than that of the polyethylene linker compound **20b**. The preliminary structure-activity relationship studies revealed that compounds with a linker containing a *meta*-substituent benzene ring showed potent neurite outgrowth-promoting activity. Altogether, compounds **20b** and **23b** exhibited remarkable neurite outgrowth-promoting activity.

We also tested Compounds **20b** and **23b** at different concentrations and incubation times (Figs. 21 (C) and 21(D)). Both compounds promoted neurite outgrowth in the range of 0.02 - 2 *µ*M (Fig. 21 (C)), and both increased the number of neurite cells in a time-dependent manner when applied at 0.02 *µ*M (Fig. 21(D)). Additionally, we examined the effect of Compound **26**, an E3 ligand, on neurite outgrowth activity (Fig. 21 (C)). Importantly, Compound **26** did not show strong neurite outgrowth activity relative to Compounds **20b** and **23b**. These findings suggested that KDM5 degradation *via* the PROTAC compounds resulted in strong neurite outgrowth.

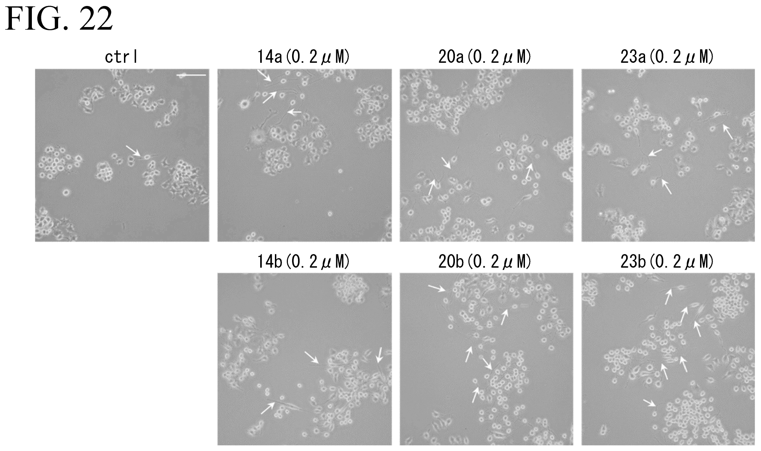

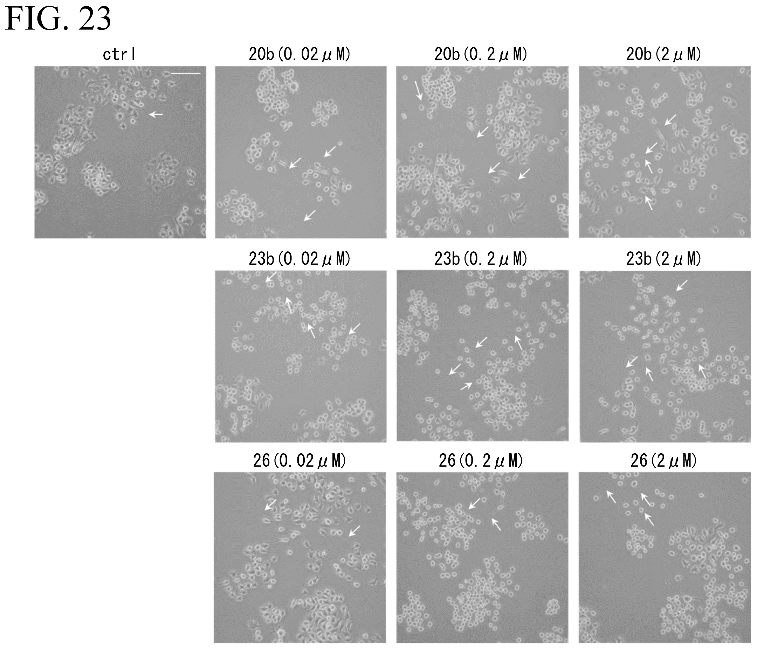

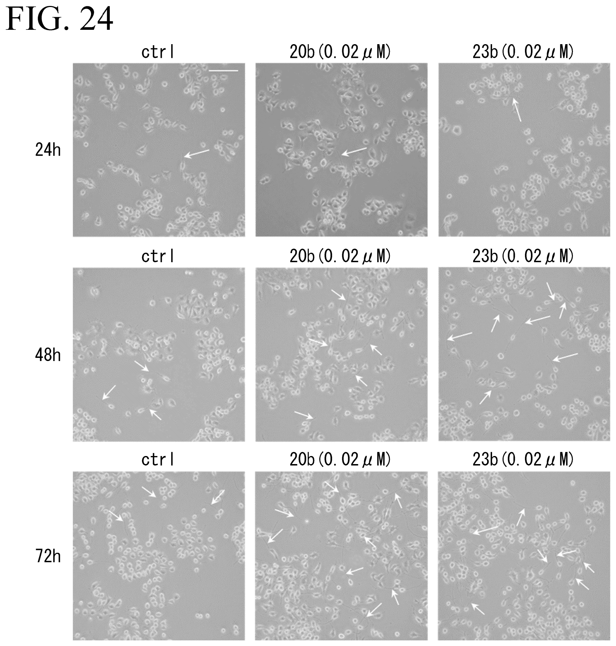

Fig. 21 (A) shows the design of KDM5 PROTAC; Fig. 21 (B) shows the effect of Compounds 14a, 14b, 20a, 20b, 23a, and 23b at 0.2 *µ*M on N2a differentiation after 48 h treatment; Fig. 21(C) shows the dose-dependent N2a cell-neurite outgrowth activities of Compounds **20b**, **23b**, and **26** after 48 h treatment; and Fig. 21(D) shows the time-dependent N2a cell-neurite outgrowth activities of Compounds **20b** and **23b** at 0.02 *µ*M. Bars represent the mean values ± S.D. from three independent experiments. For representative images showing N2a cells treated with the tested compounds, see Figs. 22-24.

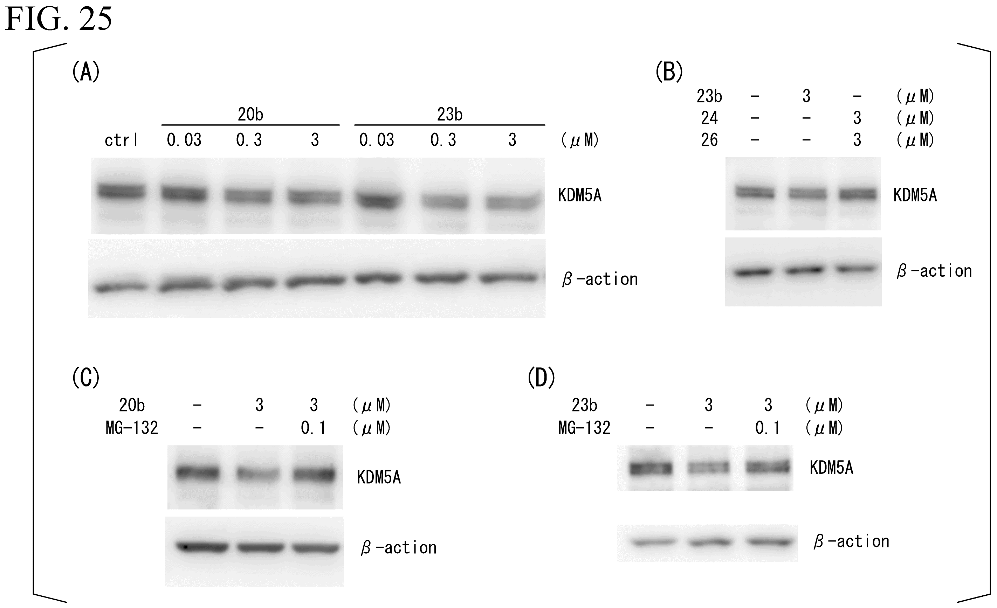

Subsequently, we tested the KDM5A degradation activity of Compounds **20b** and **23b** by means of Western blotting analysis to confirm that they work as KDM5 PROTACs. Treatment of N2a cells with Compounds **20b** and **23b** decreased KDM5A levels in a dose-dependent manner (Fig. 25 (A)). The combination of Compounds **24** and **26**, which are parent compounds of Compounds **20b** and **23b**, did not reduce KDM5A levels (Fig. 25(B)). Additionally, the decrease in KDM5A levels by Compounds **20b** and **23b** was disturbed by the proteasome inhibitor MG-132 (Figs. 25(C) and 25(D)). These results suggested that Compounds **20b** and **23b** work as PROTACs and reduce KDM5A levels in N2a cells.

Fig. 25 shows Western Blot-Detection of KDM5A Levels in N2a Cells: Fig.25(A) shows detection after 24 h treatment with Compounds **20b** and **23b;**Fig. 25(B) shows detection after 24 h treatment with Compound **23b** and co-treatment with Compounds **24** and **26**; Fig. 25(C) shows detection after 24 h co-treatment of Compound **20b** with the proteasome inhibitor MG-132; and Fig.25(D) shows detection after 24 h co-treatment of Compound **23b** with the proteasome inhibitor MG-132.

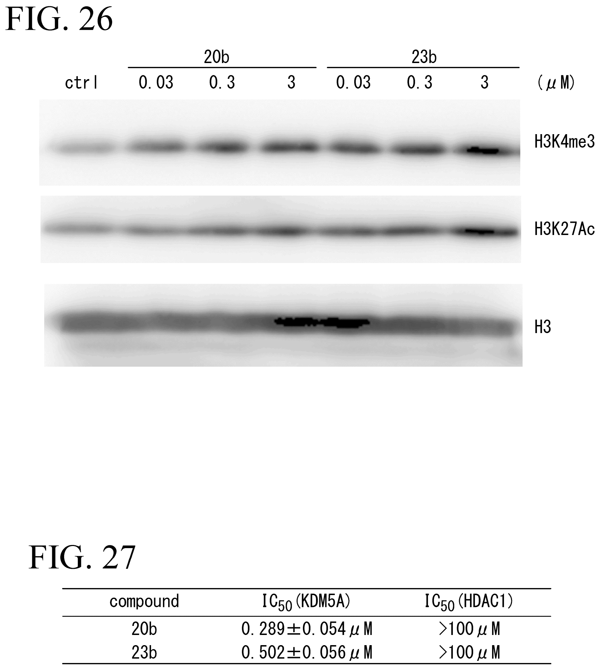

Finally, we investigated the influence of PROTAC Compounds **20b** and **23b** on histone methylation and acetylation. Because H3K4me3 is a substrate of KDM5s, we analyzed the methylation levels of H3K4 in N2a cells treated with the KDM5 PROTACs. As shown in Fig. 26, treatment with **20b** and **23b** resulted in dose-dependent accumulation of H3K4me3 levels, suggesting that **20b** and **23b** inhibited the catalytic function of KDM5s in N2a cells. Thereafter, we tested if Compounds **20b** and **23b** affected the levels of acetylated lysine 27 in histone H3 (H3K27Ac), which is both a direct substrate of HDAC1 and 2 and an indirect substrate of KDM5s, through their interaction with HDAC1 and 2. As expected, **20b** and **23b** also upregulated H3K27Ac levels (Fig. 26). Incidentally, Compounds **20b** and **23b** strongly inhibited KDM5A without affecting HDAC1 in *in vitro* assays (Fig. 27). Here, the HDAC inhibitor vorinostat (25) was used as a positive control when evaluating inhibitory activity on HDAC1, and its inhibitory activity was 99% inhibition at 1 µM. These results indicate that the degradation of KDM5A by **20b** and **23b** inhibited both the catalytic function of KDM5s to regulate H3K4me3 and the scaffolding function of KDM5s to disturb the deacetylation activity of HDAC1 and 2 in N2a cells.

Fig. 26 shows Western Blot-Detection of H3K4me3 and H3K27Ac Levels in N2a Cells after 24 h Treatment with Compounds **20b** and **23b**

### (5) Conclusion

In this study, we found that conventional KDM5 inhibitor **24** did not result in strong neurite outgrowth activity of N2a cells, although it has been reported that the inhibition of KDM5s might be effective for neurodegenerative disorders. Meanwhile, the combination of Compound **24** with the HDAC inhibitor vorinostat (**25**) significantly induced neurite outgrowth of N2a cells, implying that inhibiting both the catalytic activity of KDM5 and its scaffolding connection to HDACs affects neurite outgrowth. In this context, we focused on KDM5 PROTAC that can inhibit both these functions, and we synthesized Compounds **14a, 14b, 20a, 20b, 23a,** and **23b** as KDM5 PROTAC candidates. Among them, Compounds **20b** and **23b** significantly promoted neurite outgrowth of N2a cells. Further biological assays suggested that treatment of N2a cells with Compounds **20b** and **23b** degraded KDM5A through PROTAC-mediated proteasomal degradation. Because this degradation affected the entirety of KDM5 functionality, as opposed to traditional KDM5 inhibitors, we believe that KDM5 PROTACs are promising drugs for the treatment of neurological disorders.

### INDUSTRIAL APPLICABILITY

The present compound exhibits remarkable pharmacological effects, such as anticancer activity, in cell and animal studies.

## Claims

1. A compound represented by the following formula (I): 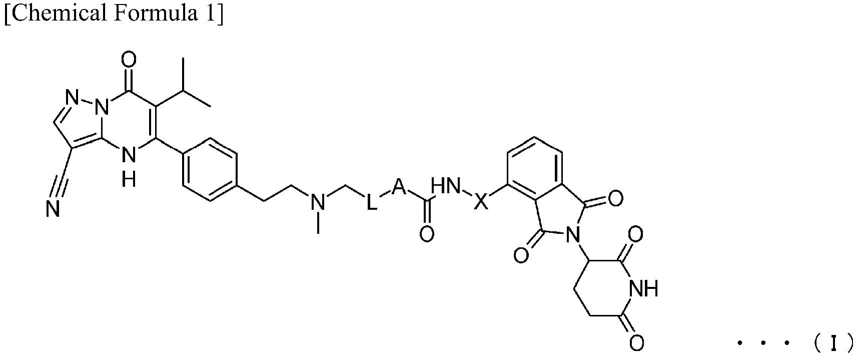 or a salt thereof, wherein:
A is a single bond or a substituted or unsubstituted divalent aromatic hydrocarbon group;
L is a substituted or unsubstituted linear or branched alkylene group having 1 to 13 carbon atoms; and
X is -(CH₂CH₂O)ₛ-, -(CH₂CH₂CH₂O)ₛ-, -(CH(CH₃)CH₂O)ₛ-, or -(CH₂)ₜO-, wherein s is a number from 1 to 8 and t is a number from 2 to 10.

2. The compound or a salt thereof according to claim 1, wherein, in said formula (I):
A is a single bond or a phenylene group;
L is a linear alkylene group having 3 to 10 carbon atoms; and
X is -(CH₂CH₂O)ₛ-, -(CH₂CH₂CH₂O)ₛ-, -(CH(CH₃)CH₂O)ₛ-, or -(CH₂)ₜO-, wherein s is a number from 1 to 5 and t is a number from 2 to 10.

3. The compound or salt thereof according to claim 1, wherein, in said formula (I):
A is a single bond, p-phenylene group, or m-phenylene group;
L is a linear alkylene group having 3 to 10 carbon atoms; and
X is -(CH₂CH₂O)ₛ-, -(CH₂CH₂CH₂O)ₛ-, or -(CH₂)ₜO-, wherein s is a number from 1 to 5 and t is a number from 2 to 10.

4. The compound or salt thereof according to claim 1, wherein said compound is a compound represented by the following formula (I-1), a compound represented by the following formula (I-2), or a compound represented by the following formula (I-3): 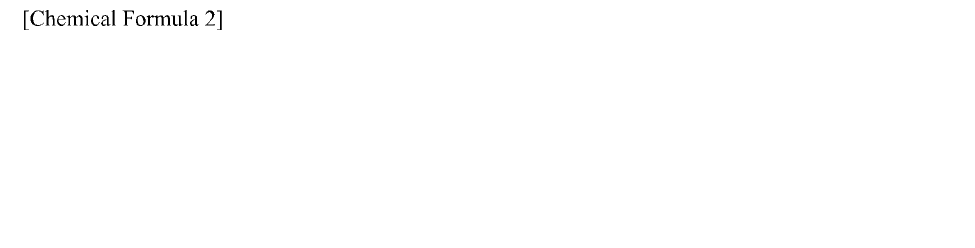 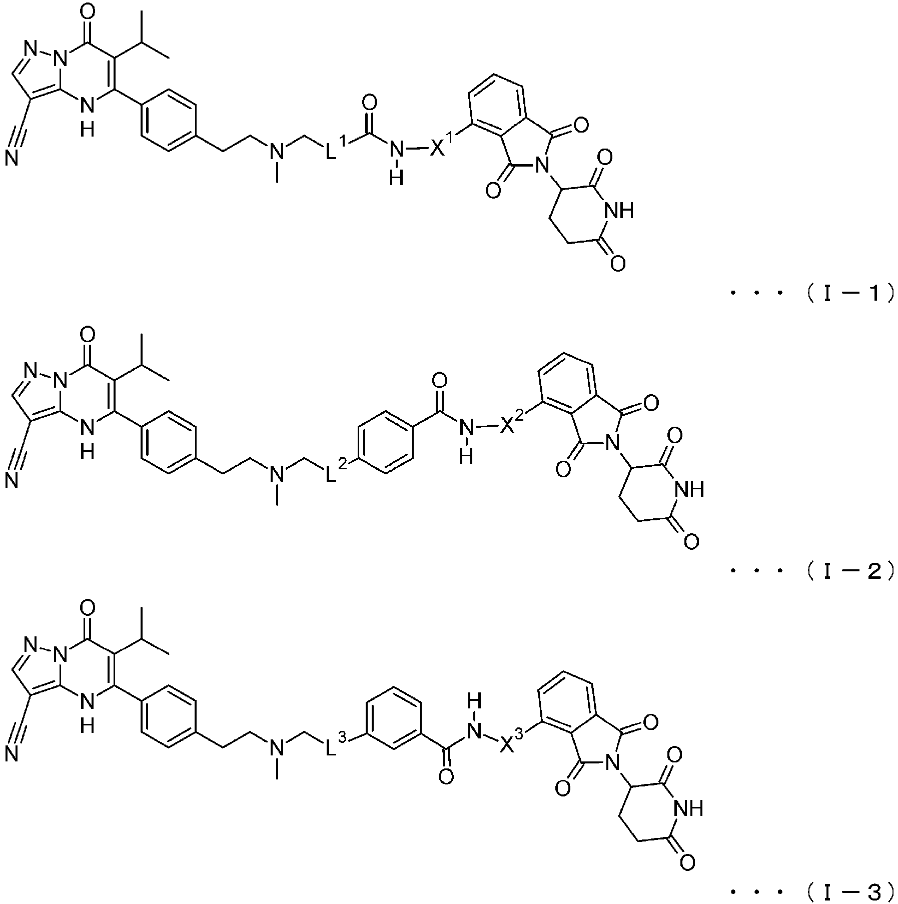 wherein:
in formula (I-1),
L¹is a linear alkylene group having 3 to 10 carbon atoms, and
X¹ is a group represented by -(CH₂CH₂O)ₛ-, wherein s is a number from 1 to 5;
in formula (I-2),
L² is a linear alkylene group having 3 to 10 carbon atoms, and
X² is a group represented by -(CH₂CH₂O)ₛ-, or -(CH₂)ₜO-, wherein s is a number from 1 to 5 and t is a number from 2 to 10; and
in formula (I-3),
L³ is a linear alkylene group having 3 to 10 carbon atoms, and
X³ is a group represented by -(CH₂CH₂O)ₛ- or -(CH₂)ₜO-, wherein s is a number from 1 to 5 and t is a number from 2 to 10.

5. The compound or salt thereof according to claim 4, wherein said compound is:
a compound of said formula (I-1), wherein L¹ is a linear alkylene group having 5 carbon atoms, and X¹ is a group represented by-(CH₂CH₂O)ₛ-, and s is 3;
a compound of said formula (I-1), wherein L¹ is a linear alkylene group having 8 carbon atoms, and X¹ is a group represented by -(CH₂CH₂O)ₛ-, and s is 3;
a compound of said formula (I-2), wherein L² is a linear alkylene group having 5 carbon atoms, and X² is a group represented by-(CH₂CH₂O)ₛ-, and s is 3;
a compound of said formula (I-2), wherein L² is a linear alkylene group having 5 carbon atoms, and X² is a group represented by -(CH₂)ₜO- and t is 6;
a compound of said formula (I-3), wherein L³ is a linear alkylene group having 5 carbon atoms, and X³ is a group represented by -(CH₂CH₂O)ₛ-, and s is 3; or
a compound of said formula (I-3), wherein L³ is a linear alkylene group having 5 carbon atoms, and X³ is a group represented by -(CH₂)ₜO-, and t is 6.

6. A pharmaceutical composition comprising the compound or salt thereof according to any one of claims 1 to 5.

7. The pharmaceutical composition according to claim 6, which is a KDM5 inhibitor and a KDM5 degrader.

8. The pharmaceutical composition according to claim 6, which is an agent for preventing and/or treating KDM5-related diseases.

9. The compound or salt according to any one of claims 1 to 5 for use in the prevention and/or treatment of KDM5-related diseases in which KDM5 inhibitory and KDM5 degrading actions are effective.

10. The pharmaceutical composition according to claim 6 for use in the prevention and/or treatment of KDM5-related diseases in which KDM5 inhibitory and KDM5 degrading actions are effective.

11. A method for preventing and/or treating a KDM5-related disease in which KDM5 inhibitory and KDM5 degrading actions are effective, the method comprising administering to a patient the compound or salt thereof according to any one of claims 1 to 5.

12. A method for preventing and/or treating a KDM5-related disease in which KDM5 inhibitory and KDM5 degrading actions are effective, the method comprising administering to a patient the pharmaceutical composition according to claim 6.

13. Use of the compound or salt thereof according to any one of claims 1 to 5 for production of a pharmaceutical composition for preventing and/or treating a KDM5-related disease in which the KDM5 inhibitory and the KDM5 degrading actions are effective.

14. An agent for preventing and/or treating KDM5-related diseases, comprising as an active ingredient a compound or a salt thereof according to any one of claims 1 to 5.
